# EUROPEAN PATENT APPLICATION

(11) **EP 1 688 435 A1**
(43) Date of publication of application: **09.08.2006**
(21) Application number: 05002131.0
(22) Date of filing: 02.02.2005
(51) Int. Cl.: C07K 16/18

(54) **Methods for determining the activity of Plk1 in human cells**

(71) Applicant: Boehringer Ingelheim International GmbH, 55216 Ingelheim am Rhein (DE)
(72) Inventor: Peters, Jan-Michael, 2103 Langenzersdorf (AT); Koch, Birgit, 1020 Wien (AT); Hauf, Silke, 71665 Vaihingen (DE); Mechtler, Karl, 2126 Ladendorf (AT); Roitinger, Elisabeth, 3051 St. Christophen (AT); Dittrich, Christina, 2000 Stockerau (AT); Peters, Beate, 2103 Langenzersdorf (AT)
(74) Representative: Hammann, Heinz

(57) **Abstract**

Antibodies recognizing the phosphorylated form of the cohesion subunits Scc1 or SA2 and methods for using such antibodies for determining Plkl activity, e.g. in clinical trials for assessing the therapeutic effect of Plkl inhibitors.

## Description

The present invention relates to the therapy of cancer with inhibitors of Polo-like kinase 1 (Plk1), in particular to methods for determining the activity of Plk1 in human cells.

The cell cycle has been recognized as a major target of therapeutic intervention in the treatment of cancer. Therefore, inhibitors that interfere with proteins of the cell cycle have gained increasing importance as anti-cancer drugs.

Anti-cancer drugs are sometimes judged for their effect on a surrogate marker (also termed "biomarker") that may predict clinical benefit to the patient, such as regression of tumors. When monitoring a patient's response to a cell cycle inhibitor, a surrogate marker is the basis for clear demonstration that an administered compound and a chosen dose and treatment schedule have reached levels sufficient to inhibit the activity of the cell cycle target.

However, traditional clinical end points have proven difficult to apply in the evaluation of molecularly targeted therapies ; standard clinical trial endpoints that are used for cytotoxic compounds have been found to be insufficient for the evaluation of molecularly targeted, e.g. cell cycle-targeted, antiproliferative agents.

In the development of molecularly targeted, e.g. cell cycle-targeted, inhibitors, it is important to have endpoint assessments available for (i) the efficacity of the compound in modulating the activity of its molecular target, and (ii) the relationship between target modulation and clinical response.

Thus, the availability of biomarkers (i.e. a measurable biological activity that indirectly indicates the effect of treatment on disease state) is one of the key prerequisites for the development of minimally invasive techniques to assess an inhibitor's efficacy and, consequently, both for the clinical evaluation of the drug and, in the long run, for monitoring the efficacy of the approved drug in therapy.

The molecularly targeted agent STI-571 (the Novartis compound also known as Gleevec) is a positive example for the contribution of a surrogate marker to successful clinical development: STI-571 was developed as a tyrosine kinase inhibitor with specificity for the Abl tyrosine kinase, although c-Kit and the platelet-derived growth factor receptor are also targets. In chronic myelogenous leukemia, 95% of patients have the Philadelphia chromosome that results in generation of the oncogenic Bcr-Abl fusion protein. Studies have shown that the activity of this oncogenic kinase is sufficient to cause the disease, thus allowing therapeutic efforts to be focused on a highly appropriate patient population (Druker and Lydon, 2000). Inhibition of tyrosine kinase activity was readily assayed by the analysis of levels ofphospho-CrkL, the predominant Bcr-Abl substrate in neutrophils. This endpoint assay assisted in the identification of a biologically active dose. The rapid and successful clinical development of Gleevec largely rests on the fact that the disease in the patient population tested was dependent on ABL activity, and there were methods available for the determination of ABL inhibition and disease remission in patients.

Another example of a cell cycle target whose activity can be readily measured is CDK4; the assay employing detection of phosphorylation, by CDK4 on pRb (Fry, et al., 2001). Western blot analysis of lysates prepared from compound-treated cells were performed using an antibody that detected phosphorylation of pRb at serine 780, a site that is thought to be phosphorylated by CDK4.

There are many examples of substrate phosphorylation sites that can serve as useful readouts for cellular inhibition of a particular molecular target. With reference to the cell cycle in particular, phosphorylation on serine 216 of the dual specificity phosphatase Cdc25C has been used to monitor the cellular activity of the Chk1 cell cycle checkpoint kinase (Graves, et al., 2000).

Furthermore, Wee1 activity has been examined by detection of phosphorylation on tyrosine 15 and threonine 14 on CDK1 using phosphospecific antibodies to these sites (Wang, et al., 2001).

Most importantly, total substrate levels as well as the level of phosphorylation on a particular site should always be determined to monitor specific signal loss (Fry, et al.,2001).

Plk 1 is another cell cycle kinase that has been considered as a target for anti-cancer therapy. This is due to increasing evidence that Plk1 forms part of the regulatory circuit that controls entry into and progression through mitosis. Plk1 belongs to the family of serine/threonine kinases, it has been shown to be strongly involved in spindle formation and chromosome segregation during mitosis. The essential role of Plk1 homologues in controlling the progression of dividing cells through mitosis has been demonstrated by genetic means in several model organisms (e.g., Drosophila melanogaster, Xenopus laevis, yeast) as well as with antibody injection or siRNA knock-down experiments in human tumor cells (Llamazares et al., 1991; Kitada et al., 1993; Lane and Nigg, 1996; Spänkuch-Schmitt, et al., 2002; Liu and Erikson, 2002; Liu and Erikson, 2003; Sumara et al., 2004). It has also been shown that Plk1 mRNA expression correlates with the mitotic activity of cells and the prognosis of lung cancer patients (Yuan, et al., 1997). Inhibitors of Plk1 are therefore thought to be promising drugs for the treatment of neoplastic diseases. However, to date no biomarkers and, consequently, no assays are available to directly monitor the effect of such a drug on the cellular activity of Plkl. Thus, in order to determine the in vivo efficacy of compounds that inhibit the protein kinase activity ofPlk1 in vitro, there is a need for biomarkers to assess P1k1 activity.

It was an object of the invention to provide reliable biomarkers for assessing P1k1 activity and, in particular, for assessing a compound's effect on the activity of Plkl.

The solution of the problem underlying the invention is based on the consideration that a desirable biomarker would be one or more protein phosphorylation sites whose generation depends on Plk1 activity. Once such sites are known, specific antibodies could be raised against them and could be used in different immunological assays to determine the presence or absence of Plk1 activity in cells and tissues.

At present, only very few phosphorylation sites are known whose generation is thought to depend on Plk1 (reviewed in Barr et al., 2004), but none of these have been rigorously validated as sites specifically modified by Plk1 in vivo.

In mitosis, the cohesin complex is phosphorylated on its subunits Scc1 and SA2. In whole cell extracts of Xenopus eggs, which recapitulate cell cycle reactions in a physiological manner, these phosphorylation reactions depend on the Xenopus ortholog ofPlk1, called Plx1 (Sumara et al., 2002). Both in Xenopus egg extracts and in living human cells the dissociation of cohesin from chromosomes depends on Plx1 and Plk1, respectively (Sumara et al., 2002; Losada et al., 2002; Giménez-Abian et al., 2004).

Based on these earlier findings, the inventors sought to determine whether phosphorylation sites on Scc1 or SA2 would be useful as biomarkers for Plk1 activity. Thus, to solve the problem of the invention, the molecular mechanisms of Plk1 activity were further analyzed:

To test whether phosphorylation of cohesin is required for its dissociation from chromosome arms during prophase and prometaphase, and to address if this modification could explain the requirement for P1k1 in this process, the inventors have searched for phosphorylation sites on all four subunits of the SA2 containing cohesin complex by mass spectrometry. The inventors have identified numerous mitosis-specific sites on Scc1 and SA2, have mutated these, and have expressed wild type and non-phosphorylatable forms of both proteins stably at physiological levels in cultured human cells. The analysis of these cell lines, in conjunction with biochemical experiments in vitro, implies that Scc1 phosphorylation is dispensable for cohesin dissociation from chromosomes in early mitosis but enhances the cleavability of Scc1 by separase at anaphase onset. In contrast, the obtained data reveal that phosphorylation of SA2 is essential for cohesin dissociation during prophase and prometaphase, but is not required for cohesin cleavage by separase. The similarity of the phenotype that is obtained after expression of non-phosphorylatable SA2 (as shown in the experiments of the invention) with the phenotype that is seen after the depletion of Plk1 (Giménez-Abian et al., 2004) strongly suggests that SA2 is the critical target of Plk1 in the cohesin dissociation pathway.

In summary, the results obtained in the experiments of the invention indicate that phosphorylation of Scc1 and SA2 in human cells depends on Plk1 and that the identified phosphorylation sites can therefore be used as biomarkers for monitoring Plkl activity.

Hence, based on the identification of phosphorylation sites of critical Plkl targets, antibodies can be provided that bind specifically to the identified phosphorylation sites and that are useful in assays for assessing Scc1 and/or SA2 phosphorylation, said phosphorylation being indicative of Plk1 activity, in particular, of a compound's effect on Plk1 activity.

In a first aspect, the present invention provides antibodies which are specifically immunoreactive with a phosphorylated form of Scc1 (SEQ ID NO:1) or SA2 (SEQ ID NO:2).

According to this aspect, the present invention relates to an antibody that binds to a phosphorylation site of the human cohesin subunit Scc1 (SEQ ID NO:1) or of the human cohesin subunit SA2 (SEQ ID NO:2) when said phosphorylation site of Scc1 or SA2 is phosphorylated and that does not bind to said potential phosphorylation site when said phosphorylation site is not phosphorylated.

In a preferred embodiment, said antibody binds to a phosphorylation site selected from serine 46, serine 138, threonine 144, serine 153, serine 175, threonine 312, serine 454, serine 459 or serine 466 of SEQ ID NO:1.

In another embodiment, said antibody binds to a phosphorylation site selected from serine 185, threonine 186, threonine 187, threonine 188 and serine 189 of SEQ ID NO:1.

In another preferred embodiment, said antibody binds to a phosphorylation site selected from serine 843, serine 1058, threonine 1118, threonine 1146, threonine 1151, threonine 1193 or serine 1224 of SEQ ID NO:2.

In another embodiment, said antibody binds to a phosphorylation site selected from serine 377 and threonine 379 of SEQ ID NO:2.

In another embodiment, said antibody binds to a phosphorylation site selected from serine 1064 and serine 1065 of SEQ ID NO:2.

In another embodiment, said antibody binds to a phosphorylation site selected from threonine 1109 and threonine 1112 of SEQ ID NO:2.

In another embodiment, said antibody binds to a phosphorylation site selected from serine 1123 and threonine 1124 of SEQ ID NO:2.

In another embodiment, said antibody binds to a phosphorylation site selected from serine 1137 and serine 1140 of SEQ ID NO:2.

In another embodiment, said antibody binds to a phosphorylation site selected from threonine 1160 and serine 1161 of SEQ ID NO:2.

In another embodiment, said antibody binds to a phosphorylation site selected from serine 1177 and serine 1178 of SEQ ID NO:2.

Preferably, the antibody recognizes a phosphorylation site that is specific for mitotic cells. Such phosphorylation sites are apparent from Table 1; phosphorylation sites with + in mitosis and no + in S-phase are preferred, sites with ++ in mitosis and no + in S-phase are particularly preferred.

The term "antibodies" as used herein, refers to immunoglobulin molecules and immunologically active portions of immunoglobulin molecules, i.e., molecules that contain an antigen binding site or paratope. Examples of such antibody molecules are intact immunoglobulin molecules, substantially intact immunoglobulin molecules and those portions of an immunoglobulin molecule that contain the paratope, including those portions known in the art as Fab, Fab', F(ab')₂ and F(v).

The antibody of the invention may be a polyclonal or monoclonal antibody, preferably, it is monoclonal.

As stated above, an antibody of the invention immunoreacts with an epitopic site of phosphorylated Scc1 or SA2. The term "epitopic site" refers to an antigenic determinant that consists of chemically active surface groupings of molecules such as amino acids or sugar side chains and usually have specific three dimensional structural characteristics, as well as specific charge characteristics. The preferred peptides containing an ectopic site and thus used for immunization to obtain the antibodies of the invention are Scc1 or SA2 peptide fragments consisting essentially of about 9 to 21, preferably about 11 to about 17 amino acid residues, wherein about 4 to 10, preferably about 5 to about 8 amino acid residues are positioned on each side of the phosphorylation site.

For generation of the antibodies of the invention, standard procedures for generating antibodies, in particular phospho-specific antibodies, can be used (Wisdom, 1994).

To obtain phospho-specific anti-Sccl and/or SA2 antibodies, a series of epitopic Scc1 and/or SA2 peptide fragments containing the respective phosphorylation sites of Scc1 and/or SA2, respectively, with a minimum length of six amino acids, usually about 9 to 21, preferably of about 11 to 17 amino acids, are synthesized.

The peptides may be synthesized such that they have a cysteine attached at the C-terminus to permit the unidirectional attachment to a carrier protein through a connecting bridge. Standard methods of conjugation also include the glutaraldehyde method which couples primarily through the α-amino group and ε-amino group, and the Maleimidobenzoyl-N-hydroxysuccinimide ester (MBS) method which couples through sulfhydryl groups. The carrier proteins most commonly used are Keyhole Limpet Hemocyanin (KLH), Bovine Serum Albumin (BSA), Rabbit Serum Albumin (RSA), Ovalbumin or Thyroglobulin (THY).

Synthesis of the phosphorylated peptides and, for specificity control and for affinity purification in the case of polyclonal antibodies, of unphosphorylated peptides, can be done according to standard procedures as described by Wisdom, 1994, e.g. by the standard Merrifield (Boc) solid phase synthesis. Methods suitable for synthesizing the phospho-peptides for immunization and of the ultimately resulting antibodies of the invention are also described by Wisdom, 1994, and inter alia, for serine-phosphorylated antibodies by Lewis at al., 1994, and for tyrosine-phosphorylated antibodies in US 5,599,681.

Another method suitable for generating the antibodies of the invention is described in US 6,309,863, which suggests a method for producing polyclonal or monoclonal phospho-specific antibodies by using for immunization a peptide containing a phosphopeptide mimetic.

The invention also provides an immunogenic composition comprising an antigenic phospho-peptide derived from a polypeptide selected from SEQ ID NO:1 or SEQ ID,N0:2, as described above.

For generating polyclonal antibodies, animals, usually rabbits, e.g. New Zealand White rabbits, are immunized with the synthetic phosphopeptide (conjugate). IgG from best responding animals, as determined by enzyme-linked immunosorbent assay against the phosphopeptide and corresponding unphosphorylated peptide, is purified by standard methods, e.g. as described in Rena et al., 1999, for the generation of generation of phosphospecific antibodies for FKHR (Forkhead transcription factors FOXO1). In brief, by this method antibodies reactive with the unphosphorylated peptide are removed by adsorption using an affinity column loaded with the corresponding unphosphorylated peptide, followed by affinity chromatography, e.g. on CH-Sepharose to which the corresponding phosphopeptide antigen had been covalently attached, and elution of the phospho-specific antibodies.

Preferably, the antibodies of the invention are monoclonal antibodies that bind specifically to the predetermined phosphoprotein epitope. Monoclonal antibody secreting hybridomas are produced using the phospho-peptides in conventional techniques (see e.g., Harlow and Lane, 1988). Phospho-specific monoclonal antibodies are usually produced by murine hybridomas formed by fusion of:
a) a mouse myeloma or hybridoma which does not secrete antibody with,
b) a murine spleen cell which secretes antibodies, obtained from a mouse immunized with a mimetic-containing polypeptide antigen.

Typically, several mice are immunized with a primary injection of antigen followed by a number of boosting injections. During or after the immunization procedure, sera from the mice are screened to identify mice which have mounted a substantial immune response. For selected mice, the spleen cells are obtained and fusions are performed. Suitable fusion techniques include, for example, the Sendai virus technique (Kohler and Milstein, 1975), or the polyethylene glycol method (Kennet, 1980).

The resulting hybridomas are then screened for production of antibodies specific for the antigen. Suitable screening techniquse are a solid phase radioimmunoassay or ELISA techniques, e.g. coating 96-well plates with phospho- or nonphosphopeptides, incubating with hybridoma supernatant and then using a labeled secondary antibody, e.g. a horseradish peroxidase-conjugated antibody, for detection. Using these technique, a solid phase is prepared by coupling the appropriate antigen to an insoluble matrix. The immunoadsorbent is brought into contact with culture supernatants of hybridomas. After a period of incubation, the solid phase is separated from the supernatants, then contacted with a labeled antibody against murine immunoglobulin. Label associated with the immunoadsorbent indicates the presence of hybridoma products reactive to the antigen.

According to traditional methods, the monoclonal antibodies are produced in large quantities by injecting antibody producing hybridomas into the peritoneal cavity of mice and after an appropriate time, harvesting ascitic fluid from the mice. The thus obtained monoclonal antibodies are then isolated from the fluid.

Preferably, alternative methods are used by which the hybridomas are cultured *in vitro;* such methods have been recently developed, mainly due to animal-welfare issues:

The simplest approach for producing monoclonal antibodies *in vitro* is to grow the hybridoma cultures in batches and purify the monoclonal antibodies from the culture medium. Serum-free media specifically formulated to support the growth of hybridoma cell lines are commercially available. After adaptation of the cells to serum-free media, cell cultures are allowed to incubate in commonly used tissue-culture flasks under standard growth conditions; the monoclonal antibody is then harvested from the medium.

To increase cell viability and the density at which the cells grow and thus increase monoclonal antibody concentration, methods can be used that employ spinner flasks or roller bottles, alternatively, commercially available gas-permeable bags may be used. To permit cells to grow at high densities, the use of a barrier, either a hollow fiber or a membrane, with a low-molecular-weight cutoff has been implemented in several devices for in *vitro culture* of hybridoma. In these semipermeable-membrane-based systems, the cells are isolated and the monoclonal antibodies produced in a small chamber separated by a barrier from a larger compartment that contains the culture media. This compartmentalization makes it possible to achieve monoclonal antibody concentrations comparable with those in mouse ascites. In a hollow-fiber bioreactor, the medium is continuously pumped through a circuit that consists of a hollow-fiber cartridge, gas-permeable tubing that oxygenates the media, and a medium reservoir. The hollow-fiber cartridge is composed of semipermeable multiple fibers that run through a chamber that contains hybridoma cells growing at high density. Such hollow-fiber bioreactors are commercially available, e.g. the so-called "Tecnomouse" from Integra Biosciences).

Next, the resulting antibodies are, using standard technology (e.g. Wisdom, 1994), purified and, in order to determine the extent of their phosphospecificity,
characterized by immunoassays, e.g. enzyme-linked immunosorbent assay (ELISA), for binding to the respective phospho- and non-phosphopeptides. To this end phosphorylated and non-phosphorylated peptides are coated onto 96-well microtiter plates and, after blocking unspecific binding sites with excess protein, incubated with the solution containing the antibodies to be characterized (e.g. hybridoma supernatant, dilutions of purified antibodies). After allowing the antibodies to bind, unbound antibodies are washed off and a secondary antibody directed towards either mouse IgG or rabbit IgG is added to the wells. The secondary antibody is typically conjugated with an enzyme such as horse radish peroxidase (HRP) in order to visualize the specific binding of primary antibody to the coated peptide by the addition of a suitable substrate (e.g. 2,2'-Azino-bis(3-ethylbenzothiazoline-6-sulfonic acid) diammonium salt from Sigma Aldrich)

Antibodies specific for a given phospho-epitope should recognize the phosphorylated but not the non-phosphorylated form of the peptide used. Subsequently, the antibodies of the invention are analysed by standard Western blotting technique to further determine specificity against Scc1 or SA2, respectively. To this end, whole cell lysates from human tumor cell lines are separated by SDS-PAGE and blotted onto e.g. nitrocellulose membranes. The membrane is subsequently incubated with blocking solution and then the antibody directed against the phosphoepitope(s) is added. After incubation unbound antibody is washed away and an secondary antibody conjugated e.g. with HRP (horse raddish peroxidase) is added. After washing off unbound antibodies the bound antibodies are visualized by ECL (enhanced chemiluminesce). The antibodies should give a single band at the molecular weight corresponding to Scc1 or SA2. To check the specificity of the antibodies, incubation of the membrane with the primary (e.g. phosphospecific) antibody is also done in the presence of phospho- or nonphospho- polypeptide selected from SEQ ID NO:1 or SEQ ID NO:2, as described above. The obtained signal is considered to be specific if it can be competed away with the phospho-polypeptide but not with the corresponding non-phospho-polypeptide.

To select those antibodies that distinguish most acutely between mitotically arrested and logarithmically growing cells, a lysate of mitotically arrested cells (in which Scc1 and SA2 are predominantly present in their phosphorylated state due to Plk1 activity) is contacted with a candidate antibody and the reactivity of the antibody is determined by Western blot analysis as described above. The obtained signal is compared side by side with the signal obtained with lysates of logarithmically growing cells. Antibodies which yield a strong signal in lysates from mitotically arrested cells but do only yield a very weak signal in lysates from logarithmically growing cells are best suited to be used in assays for assessing Plk1 activity.

In a further aspect, the invention also provides a method for detecting the presence of phosphorylated Scc1 and/or SA2 wherein a biological sample suspected ofPlk1 activity (and thus containing phosphorylated Scc1 and/or SA2) is contacted with a phospho-specific antibody of the invention and binding of the antibody is determined. Due to the antibody's ability to distinguish between phosphorylated and non-phosphorylated Scc1 or SA2, which are substrates of Polo-like kinase 1 (Plkl), the readout of said measurement can be directly correlated with P1k1 activity. In an embodiment of the invention, in order to determine the pharmacodynamics of a drug that acts as a Plk1 inhibitor, i.e. the action of the drug in the body over a period of time, including the processes of absorption, distribution, localisation in the tissues, biotransformation, and excretion, the activity of Plk1 is determined in a biological sample. For determining the pharmacodynamic activity of a drug that acts as a Plk1 inhibitor, the biological sample is derived from a patient who is being treated with said Plk1 inhibitor.

The sample may be any tissue or body fluid from a subject. Ideally, the sample comprises proliferating cells that are positive for cohesin, e.g. tumor cells or, alternatively, cells derived from tissue with mitotic activity, e. g. hair root cells (radix pili cells) or mouth mucosa cells.

In a further aspect, the invention relates to a method for determining a compound's effect on Plk1 activity in an individual treated with a compound by determining *ex vivo* before and after administration of said compound to said individual the presence of phosphorylated Scc1 and/or SA2 in a sample and comparing the levels of phosphorylated Scc1 and/or SA2 before and after administration of the compound, wherein a reduced level of phosphorylated Scc1 and/or SA2 upon administration of the compound is indicative of its inhibitory action on Plk1.

In a variation of this method, the compound's effect on Plkl activity may be determined over a period of time at pre-defined time points by comparing the levels of phosphorylated Scc1 and/or SA2 over said period of time, wherein a reduced level of phosphorylated Scc1 and/or SA2 is indicative of a compound's therapeutic effect.

In principle, the above described methods can be conducted by using a single antibody, e.g. an anti-SA2 antibody, however, two or more assays employing different phospho-specific anti-SA2 and/or anti-Scc1 antibodies may be done in parallel.

In the above described assays, the desired therapeutic effect of a Plk1 inhibitor should manifest itself in a decrease of phosphorylated Scc1/SA2 relative to the overall amount of Scc1/SA2.

The antibodies of the invention are also useful for determining the specificity of a compound's inhibitory effect on Plk1. To this end, the amount of phosphorylated Scc1/SA2 is compared with the level of other cell cycle biomarkers, e.g. with the level of cyclinB, phosphorylated histone H3 or MPM2-reactive signal (or other enzymes or markers expressed in mitosis). Administration of a specific Plk1 inhibitor should result in a decrease of the Plk1-specific Scc1/SA2 phosphorylation signal, while the other cell cycle markers should be unaffected by the compound. Thus, upon treatment of a patient with Plk1 inhibitor the biological sample from a tissue or fluid that normally contains proliferating cells should, at a given time point, show high levels of mitotic markers (e.g. cyclin B, MPM-2 or histon H3 phospho-signal, Ki-67, Aurora kinase etc.) and reduced or no levels of Plk1-specific Scc1/SA2 phosphorylation signal. The degree of the observed mitotic arrest and the degree of downregulation of Plk1-specific Scc1/SA2 phosphorylation signal can be used to assess the pharmacodynamic activity of the administered drug. In insights gained from this evaluation can be used to help guiding the adjustment of drug dose and treatment schedule.

The antibodies of the invention may also be used for selecting patients for treatment with Plk1 inhibitors. To this end, tumor samples from cancer patients are tested for Plk1 activity as described above. Since a high level of Scc1/SA2 phosphorylation in the tumors of patients may be a consequence of aberrant Plk1 activity, it may be expected that these patients are likely to benefit from treatment with a P1k1 inhibitor.

The methods of the invention for determining Scc1 and/or SA2 phosphorylation status can be carried out using standard immunohistochemical methods. To this end the collected patient sample is prepared for immonohistochemical analysis as it has been described previously (e.g by Harlow and Lane, supra). Briefly, the patient sample containing proliferating cells is embedded into an appropriate matrix (e.g. paraffin or TissueTec for cryopreservation) or smears or spreads are prepared from collected cellular material. Sections from the embedded tissues are generated and are subjected to primary and secondary antibody incubations. Plk1-specific Scc1/SA2 phosphorylation signal or proliferation markers are visualized using peroxidase staining or an equivalent technique. In addition, standard tissue staining techniques such as hematoxilin staining can be used for histological analysis (Harlow and Lane, supra). Instead of using the antibodies of present invention for immunohistochemical analysis, the antibodies may be also used for the analysis of the patient sample by Western Blot (as described above) or isolated patient cells can be subjected to FACS analysis. To this end, such isolated cells are fixed, permeabilized and incubated with primary (e.g. Scc1/SA2 phosphorylation specific antibodies and antibodies specific for other mitosis markers as described above) and secondary antibodies conjugated with fluorescent tags. The signals for Scc1/SA2 phosphorylation status and the signal for other mitotic markers is then determined by flow cytometry in a fluorescensce activated cell sorter (FACS).

In another aspect, the antibodies of present invention are useful in screening methods for determining or confirming a compound's ability to inhibit P1k1 in a cell.

Such screening methods can be conducted according to known principles, e.g. by adapting the method described in US 6,287,784 that has been suggested for screening kinase inhibitors by determining autophosphorylation of kinase receptors. In an assay for identifying Plk1 inhibitors, by way of example, a first solid phase is coated with a substantially homogeneous population of cells having Plk1 activity, e.g. proliferating cells that are arrested/synchronized in mitosis (e.g. by incubating them with nocodazole), so that the cells adhere to the first solid phase. Following exposure to the candidate compounds for a period of time sufficient for the compound to exert its inhibitory effect, e.g. for 5 minutes to 5 hours, the adherent cells are solubilized, thereby releasing cell lysate. A second solid phase is coated with a capture antibody that binds to Scc1 or SA2 at a site different from a phosphorylation site. The cell lysate obtained from the first solid support is added to the second solid support containing the adhering capture antibody in order to capture Scc1 or SA2, respectively. A washing step is then carried out to remove unbound cell lysate, leaving the captured substrate bound to the second solid support. Next, the captured Scc1 or SA2 is exposed to a labelled antibody of the invention, which identifies a phosphorylated residue in Scc 1 or SA2. In a final step, binding of the phospho-antibody to the captured substrate is measured. The obtained signal is compared with the signal obtained from control cells without exposure of compound.

### Brief description of the figures:

- Figure 1:: Identification of mitosis-specific phosphorylation sites on human cohesin.
- Figure 2:: Plkl facilitates cleavage of human Scc1 by separase in vitro.
- Figure 3:: Characterization of HeLa cell lines stably expressing wild-type or mutant forms of human Scc1 and SA2.
- Figure 4:: Mutations in the C-terminal putative phosphorylation sites and C-terminal deletions abolish mitotic phosphorylation of SA2.
- Figure 5:: Phosphorylation of SA2 is required for cohesin dissociation from chromosome arms during pro- and prometaphase.
- Figure 6:: The presence of SA2 12xA on chromosome arms correlates with cohesion between sister chromatid arms.
- Figure 7:: Phosphorylation of SA2 is required for efficient resolution of sister chromatid arms during prometa- and metaphase.
- Figure 8:: Mitosis-specific phosphorylation sites in human Scc1 and SA2.
- Figure 9:: Phosphorylation of Scc1 is not required for dissociation of cohesin from chromosome arms during prometa- and metaphase.
- Figure 10:: Condensation or condensin binding is not impaired in SA2 12xA expressing cells.
- Figure 11.: Antibodies raised against mitosis specific phosphorylation sites on the cohesin subunits Scc1 and SA2 only react with cohesin isolated from mitotic cells.
- Figure 12.: Antibodies raised against mitosis specific phosphorylation sites on the cohesin subunits Scc1 and SA2 can react with cohesin isolated from interphase cells after phosphorylation of cohesin by Plk1.
- Figure 13.: The reactivity of phospho-specific antibodies with Scc1 is abolished by mutation of the phosphorylation site against which the antibodies were raised.

In the following Examples, if not otherwise stated, the following materials and methods were used:

### Cell lines and growth conditions

HeLa cells expressing myc-tagged human Scc1 were described previously(Hauf et al., 2001). HeLa cells expressing mutated Sccl, wild-type or mutated SA2 were generated by transfecting HeLa tet-on cells (Clontech) with pTRE2-hygro vector (Clontech) containing the respective cDNA and a 9xmyc-tag fused in frame.

Hygromycin-resistant clones were selected by growth in medium containing 400 µg/ml Hygromycin, and were tested for expression of the myc-tagged protein by immunoblotting after induction with 2 µg/ml Doxycycline for 1 to 2 days. Untransfected HeLa cells were grown in DMEM supplemented with 10 % FCS, 0.2 mM L-glutamine, 100 U/ml penicillin, and 100 µg/ml streptomycin. For growth of stably transfected HeLa tet-on cells, the medium was supplemented with 200 µg/ml G418 and 100 µg/ml Hygromycin. Hydroxyurea and Nocodazole were used at a concentration of 2 mM and 330 nM respectively.

### cDNA mutagenesis

Serine or Threonine residues in human Scc1 or SA2 were changed to Alanine by mutation of the respective cDNA using the Quick Change Site-Directed or Multi Site-Directed Mutagenesis kit (Stratagene). Where the phosphorylated site could not be assigned to a certain residue within a peptide, all possible candidate sites were mutated to Alanine. C-terminal truncated cDNAs of SA2 coding for amino acid 70 to amino acid 1056, 1107, 1135 or 1175 were generated by PCR and cloned into pcDNA 3.1 (-)/Myc-His A (Invitrogen) modified to contain a 9xmyc-cassette. For the expression of cDNAs as ³⁵S-Methionine and ³⁵S-Cysteine-labeled proteins, coupled transcription-translation reactions in rabbit reticulocyte lysate (Promega) were performed.

### Antibodies

Antibodies specific to phosphorylated threonine were from Cell Signaling Technology (catalog # 9381). To detect myc-tagged protein, either polyclonal, affinity-purifed rabbit-anti-myc antibody (Gramsch Laboratories, CM-100), or monoclonal 9E10 mouse-anti-myc antibody (Waizenegger et al., 2000) were used. Polyclonal antibodies to human Scc1, SA1, SA2, SMC1, SMC3, and SMC2 have been described (Sumara et al., 2000; Waizenegger et al., 2000; Yeong et al., 2003). Anti-INCENP peptide antibody was raised in rabbit, and the serum was affinity purified. The following peptide was used for immunization: TDQADGPREPPQSARRKRSYC.

### Mass spectrometry

Immunopurified cohesin complex was digested in solution with different proteases (trypsin, Glu-C, trypsin/Glu-C, chymotrypsin, subtilisin, elastase) according to Yates et al., 2000. Proteolytic peptides were applied to a precolumn (PepMAP C18, 0.3 x 1 mm, Dionex) and eluted onto an analytical column (PepMAP C18, 75 µm x 150 mm, Dionex). The eluted peptides were introduced via a nanospray ion source interface (Protana) into an ion trap mass spectrometer (Finnigan LCQ). The mass spectrometer cycled through five scans, one full mass scan followed by four tandem mass scans of the four most intense ions. All tandem mass spectra were searched against the human non-redundant protein database by using the SEQUEST program (Flicka). Any phosphopeptide matched by computer searching algorithms was verified manually.

### In vitro Scc1 cleavage assays

The in vitro cleavage assay was performed as described (Waizenegger et al. 2000) with the exception that in vitro translated human wild type or mutant Scc1-myc was used as substrate. To isolate human separase, purified polyclonal antibodies generated against recombinant human separase were used. In some reactions human GST-Plkl was added in a concentration of approximately 50 ng/µl.

### Immunoprecipitation

Cohesin was purified by peptide antibody affinity chromatography as described (Sumara et al. 2000). 300 to 500 µg of purified antibodies were coupled and crosslinked to Affi-prep protein A beads (BioRad) at 1 µg antibody/µl beads. Cohesin was immunoprecipitated from 3 to 5 ml HeLa extract containing approximately 10 µg protein/µl.

### Sucrose density gradients

Sucrose density gradient centrifugation was performed as described (Hauf et al., 2001).

### Mitotic mobility assay for SA2

Xenopus interphase egg extracts were supplemented with in vitro translated ³⁵S-labeled SA2, which had C-terminal deletions or mutations of phosphorylation sites. Extracts were induced to enter mitosis by addition of cyclin B Δ90 and 1 µM OA as described in Sumara et al. (Sumara et al., 2000). Samples were collected at the indicated time points and analyzed by SDS-PAGE followed by Phosphorimager analysis.

### In vitro phosphorylation assay

SA2-myc or SA2-12xA-myc containing cohesin complexes were purified by immunoprecipitation with affinity-purified rabbit-anti-myc antibody (Gramsch Laboratories, CM-100). Conditions of the in vitro phosphorylation assay have been described (Kraft et al., 2003).

### Immunofluorescence

For immunofluorescence, cells were either grown on coverslips or spun onto glass slides using a Cytospin centrifuge (Shandon). Cells were extracted with 0.1 % Triton X-100 prior to fixation to remove the soluble pool of cohesin. Fixation, immunostaining, and image acquisition were performed as described (Waizenegger et al., 2000).

### Chromosome spreads

Chromosome spreads followed by Giemsa staining were performed as described (Hauf et al., 2003).

### Quantification of interchromatid distance

On pictures of chromosome spreads, a line scan was performed across chromosome arms orthogonal to the long axis of the chromosome using MetaMorph software (Universal Imaging Corp.). On the line scan, the distance from peak to peak was measured. Five chromosomes were thus analyzed per cell, and the resulting distances were averaged. More than 50 cells were randomly picked per cell line, and thus analyzed. Each pixel on the diagram represents one cell.

### Example 1

Identification of Mitosis-Specific Phosphorylation Sites on Human Cohesin

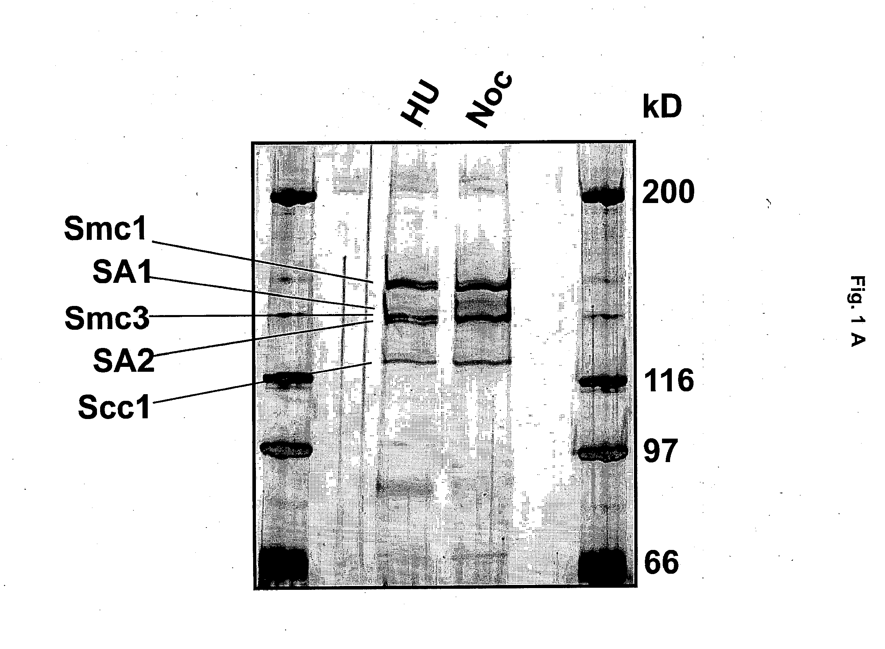

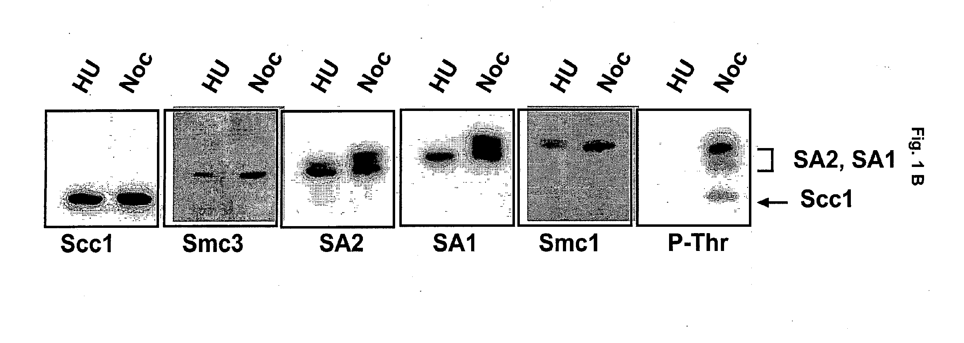

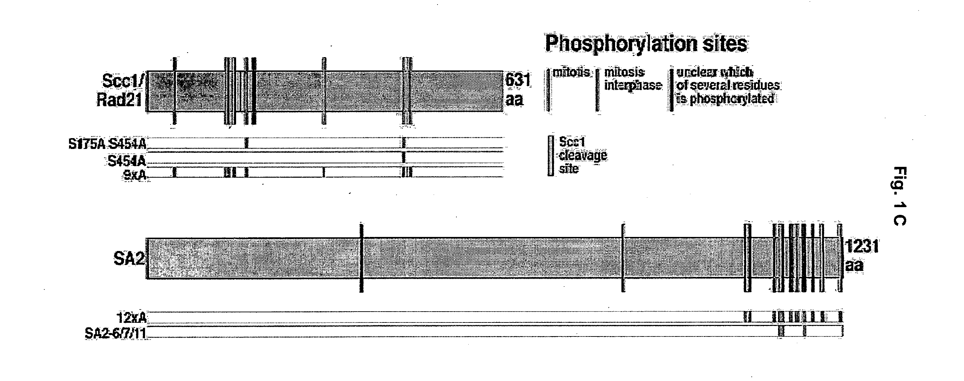

The SA1, SA2 and Scc1 subunits of vertebrate cohesin complexes are phosphorylated specifically in mitosis (Losada et al., 2000; Hoque and Ishikawa, 2001; Sumara et al., 2002). To be able to analyze the functional relevance of these modifications, the mitosis-specific phosphorylation sites on human cohesin were mapped by mass spectrometry. Lysates from HeLa cells were prepared that had been arrested either at the G1/S transition by hydroxyurea (interphase) or in mitosis by the spindle poison nocodazole, and immunoprecipitated SA1 and SA2 containing cohesin complexes with antibody 447 that recognizes both SA1 and SA2 (Sumara et al., 2000). Separation of the isolated proteins by SDS-PAGE and staining with silver (Figure 1A) or cohesin specific antibodies (Figure 1B) demonstrated that both SA1 and SA2 showed reduced electrophoretic mobility when the complexes had been isolated from mitotic cells, indicating that the mitosis specific phosphorylation of these subunits was preserved during the isolation procedure. This notion was confirmed by immunoblotting with phospho-threonine specific antibodies which yielded a pattern that was consistent with the presence of phospho-threonine residues on SA1, SA2, and Scc1 in mitosis, whereas no phospho-threonine signal was detected on cohesin subunits isolated from interphase cells (Figure 1B).

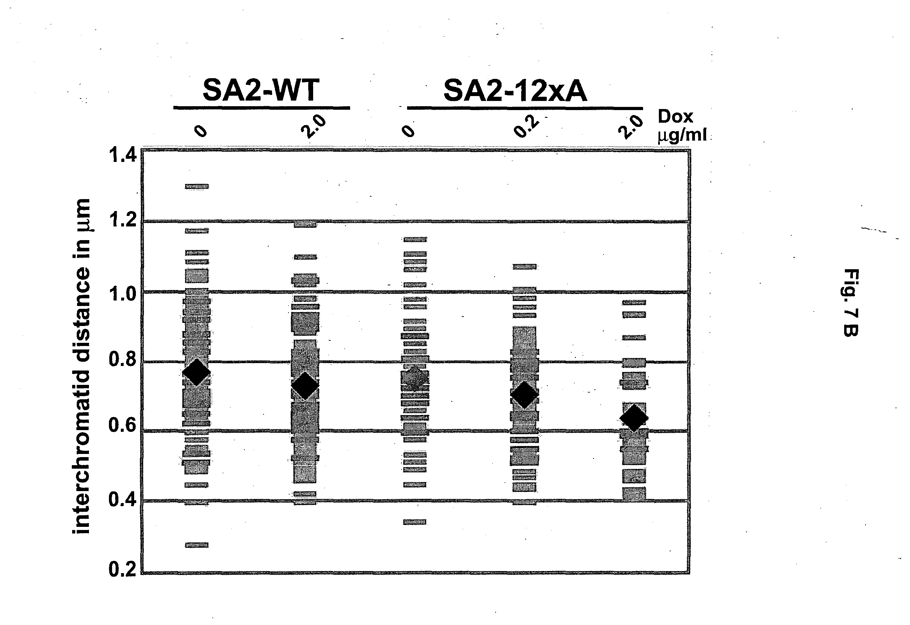

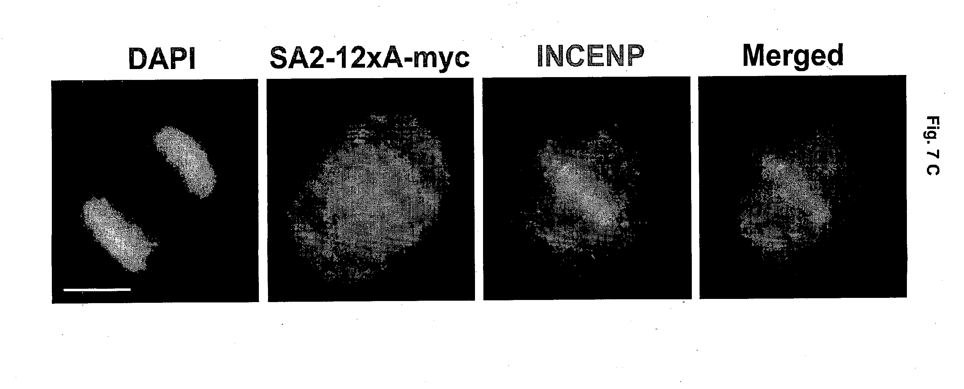

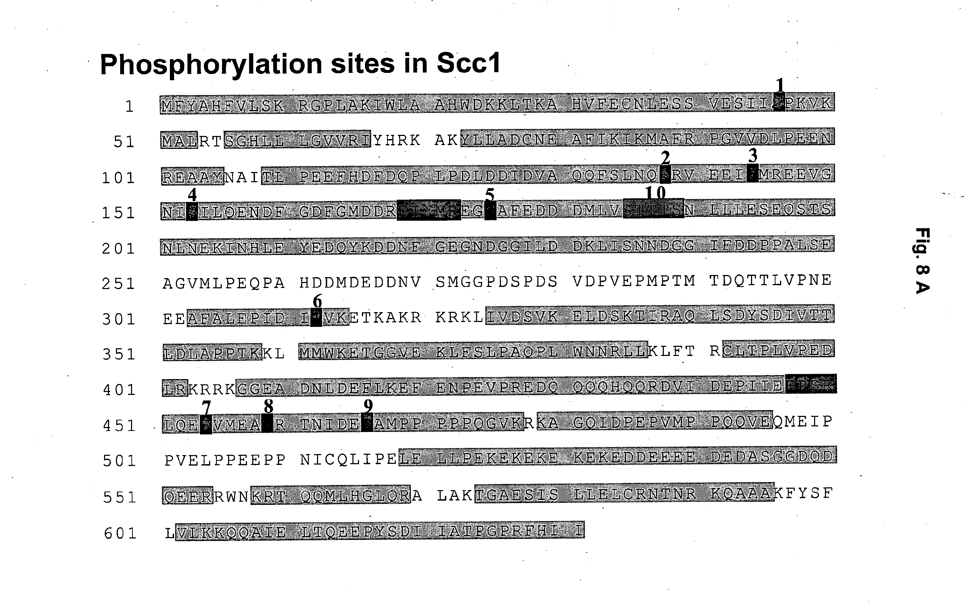

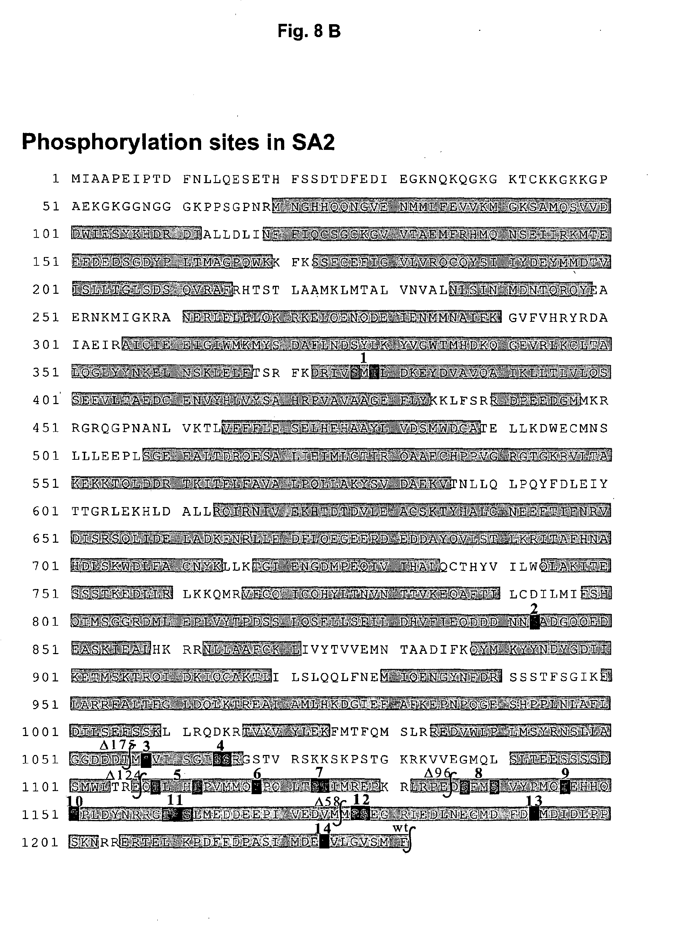

To identify phosphorylation sites, purified cohesin complexes were digested with various proteases in solution and analyzed by HPLC-ESI-MS/MS. In total, 28 phosphorylated serine or threonine residues were identified, 18 of which could be assigned unequivocally to specific residues, whereas in ten cases it needed to be confirmed which of several serine and threonine residues in a given peptide was phosphorylated (Figure 1C, Table 1). Two sites each were found in Smc1 and Smc3, 10 in Scc1 and 14 in SA2. In this experiment, it was not possible to identify phosphorylated peptides derived from SA1, presumably because SA1 containing cohesin complexes are much less abundant than SA2 containing complexes (Losada et al., 2000; Sumara et al., 2000). By analyzing cohesin peptides obtained by digestion with various proteases a sequence coverage of approximately 80 % could be obtained for each of the subunits (Figure 8) indicating that this analysis identified the majority of in vivo phosphorylation sites on cohesin subunits.

The phosphorylation sites on Smc1 and Smc3 were present in complexes from both interphase and mitotic cells, and they were therefore not analyzed them any further. Two of these sites (Ser 966 and Ser957 in Smc1) have previously been shown to be phosphorylated by ATM kinase in response to DNA damage (Kim et al., 2002; Yazdi et al., 2002). Only one of the sites in Scc1 and SA2 (S153 in Scc1) was also found phosphorylated in interphase, all other sites were only identified in cohesin samples from mitotic cells. These results confirm that Scc1 and SA1/2 are specifically phosphorylated in mitosis.

Since Plk1/Plx1 can phosphorylate Scc1 and SA1/SA2 in vitro and is required for the phosphorylation of these subunits in Xenopus egg extracts (Sumara et al., 2000), the sequence surrounding the identified sites was compared to the consensus sequences that have been reported as binding and phosphorylation sites for Plk1. The C-terminal Polo box domain of Plk1 binds to phospho-sites for which the consensus S-[pT/pS]-[P/x] has been proposed (Elia et al., 2003). These phosphosites are thought to be generated by proline directed kinases such as Cdk1, which "prime" the substrate for subsequent recognition by Plk1. Once bound to the substrate, Plk1 is thought to phosphorylate sites that are distinct from the one that is recognized by the Polo box. Based on in vitro phosphorylation experiments using peptides derived from the kinase Myt1, Nakajima et al. (Nakajima et al., 2003) have proposed the consensus [ED]-x-[ST]-φ-x-[ED] for Plk1 phospho-sites, where φ signifies a hydrophobic amino acid, whereas Barr et al. (Barr et al., 2004) have suggested the consensus [EDQ]-x-[ST]-φ. However, Plk1 has also been reported to phosphorylate sites that do not entirely conform with these consensus sequences, for example the EDpSGYpSS sequence in the APC/C inhibitor Emi1 (Moshe et al., 2004). One putative phospho-site in Scc1 (Thr186) and three putative sites in SA2 (Ser1065, Thr1024, Ser1178) match the Polo box binding consensus sequence, although none of these sites contains a proline residue at the +1 position (these are putative sites because they are present in peptides in which the exact identity of the residue carrying the phospho-moiety could not be determined; Table 1). It was also noted that, although Cdk1 can phosphorylate Scc1 and SA1/2 in vitro (Losada et al., 2000), it does not appear to be essential for cohesin dissociation in vivo (Sumara et al., 2000; Sumara et al., 2002). Two phospo-sites in Scc1 (Thr144, Thr312) match the consensus proposed by Nakajima et al. (Nakajima et al., 2003). These two sites, one additional site in Scc1 (Ser454) and three in SA2 (Thr1109, Ser1137, Ser 1124) conform with the consensus proposed by Barr et al. (Barr et al., 2004). These findings are consistent with the possibility that at least some of the sites in Scc1 and SA2 are directly phosphorylated by Plk1.

Multiple sequence alignment showed that most of the amino acid residues that were found to be phosphorylated in mitotic human Scc1 and SA2 were conserved in orthologs from other vertebrates, but not in more distantly related eukaryotes like Drosophila, C. elegans, or yeast, where the homology to human cohesin subunits is in overall much lower. When the distribution of the sites was regarded, it was found that Scc1 phosphorylation sites strikingly clustered around the two separase cleavage sites, whereas SA2 phosphorylation sites were concentrated in the C-terminus of the protein (Figure 1C). The clustering of Scc1 phosphorylation sites in the vicinity of separase cleavage sites has also been observed in budding yeast (Alexandru et al., 2001). This indicates that, although individual sites on Scc1 are not conserved from yeast to human, the overall pattern of phosphorylation is conserved, at least for Scc1. Evolutionary conservation of the overall mitotic phosphorylation pattern, but not of individual sites, has also been observed in the case of another multi-subunit complex, the APC/C (Kraft et al., 2003).

### Figure 1:

(A,B) Cohesin was immunoprecipitated by antibody 447 (recognizing SA1 and SA2) from extracts prepared from HeLa cells which were either arrested in S-phase by hydroxyurea (HU) or in mitosis by Nocodazole (Noc). Cohesin was eluted by low pH and analyzed by silver staining and immunoblotting with antibodies to cohesin subunits and phosphorylated threonine (P-Thr).
(B) Schematic representation of the phosphorylation sites on Scc1 and SA2 that were identified by mass spectrometry, and of the mutant versions that have been generated.
(C) Schematic representation of the phosphorylation sites on Scc1 and SA2 that were identified by mass spectrometry.

### Figure 8:

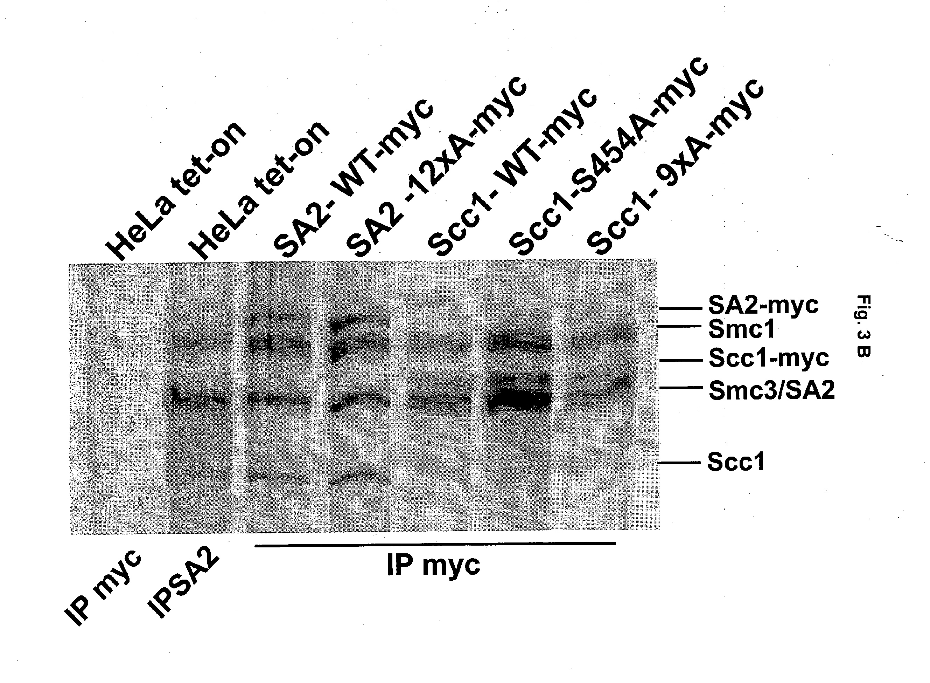

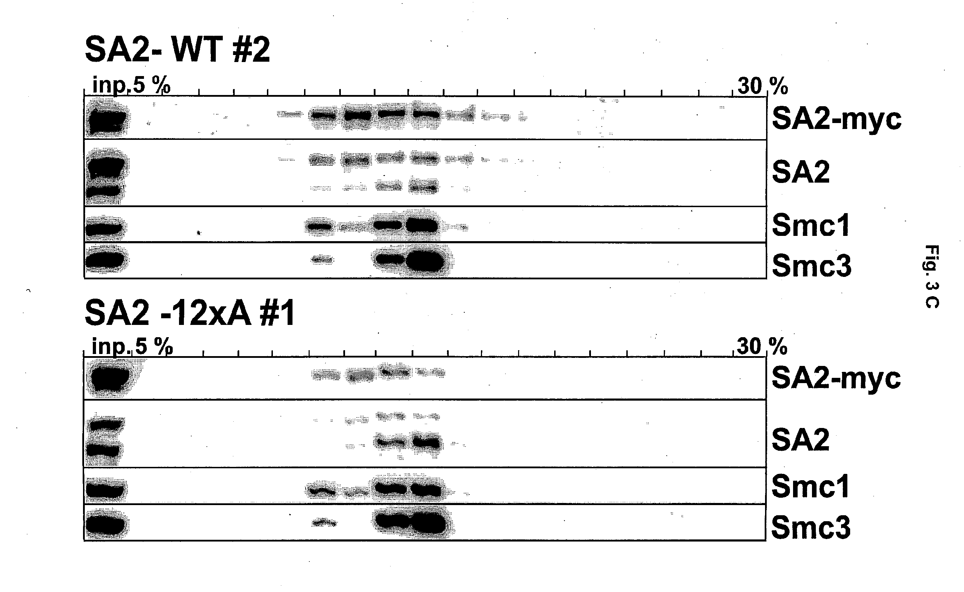

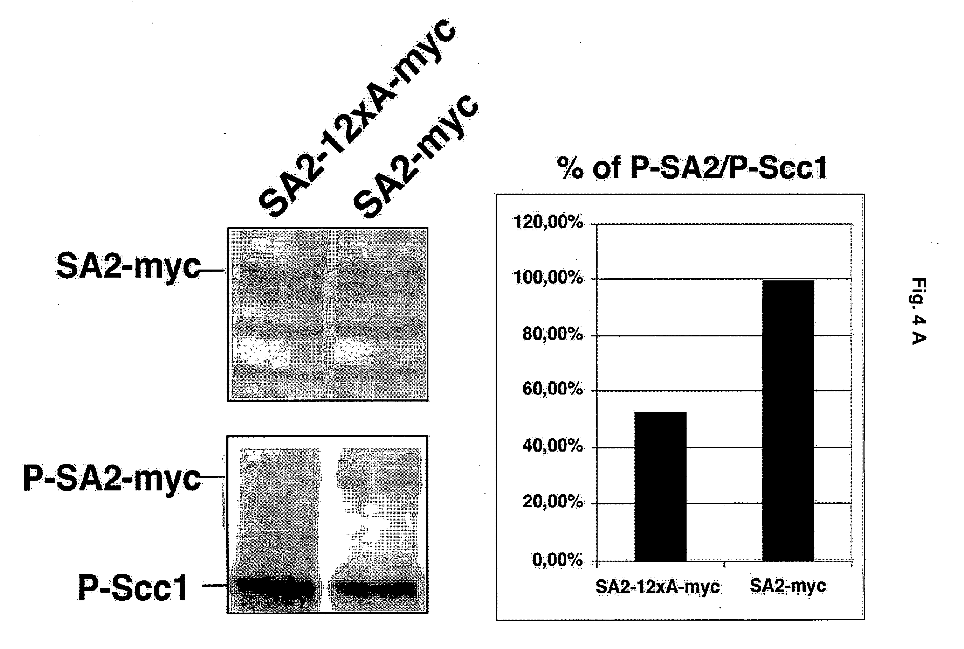

(A,B) The residues marked in red and blue were found to be phosphorylated in mitosis by mass spectrometry. Residues marked in red were unambiguously identified, whereas in the regions marked in blue the phosphorylated residue could not be assigned with certainty. The separase recognition sites on human Scc1 are indicated in green. Peptides identified by mass spectrometry are in yellow. The residues at which SA2 was truncated for the assay in Figure 4C are also indicated.

### Example 2

### Phosphorylation of Scc1 is Required for Efficient Cleavage by Separase in Vitro

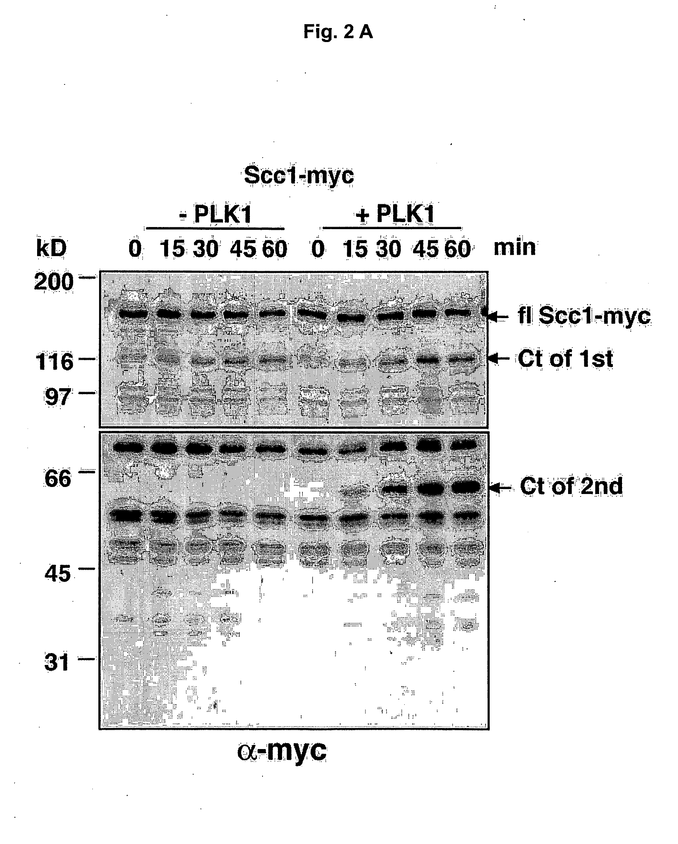

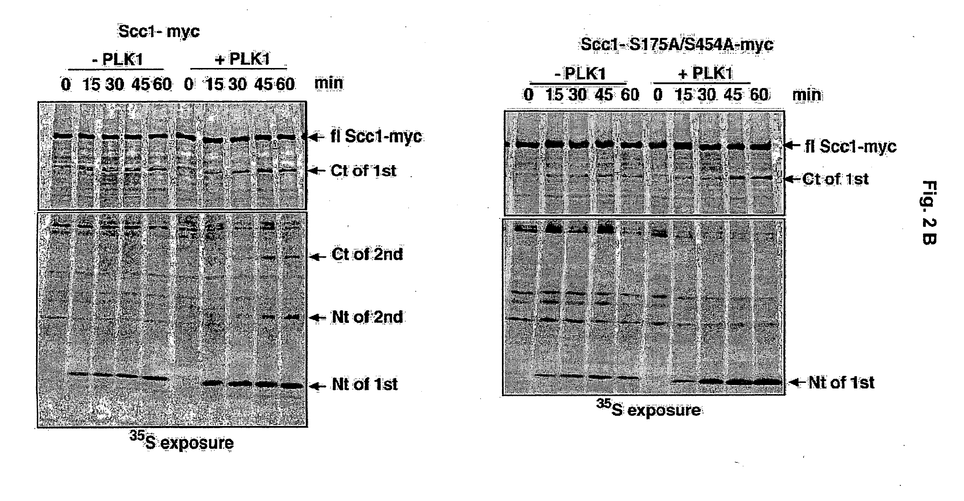

It was analyzed if treatment of human Scc1 with Plk1 increases its cleavability by separase. Purified human separase-securin complexes were activated by securin destruction in mitotic Xenopus egg extracts and incubated with ³⁵S-labeled recombinant Scc1 as a substrate. Under these conditions separase cleaves Scc1 at the same sites that are cleaved at anaphase onset in vivo, Arg172 and Arg450 (Hauf et al., 2001; Figure 2A and B). When untreated Scc1 was incubated with separase, efficient cleavage could only be detected at the first Scc1 site, resulting in the formation of one N-terminal and one C-terminal cleavage product which could be seen by Phosphorimager analysis (Figure 2B). The C-terminal cleavage product could also be detected by immunoblotting by using antibodies to a myc epitope tag on the C-terminus of Scc1 (Figure 2A). When the cleavage reaction was carried out in the presence of active recombinant Plk1, cleavage at the first Scc1 site was slightly enhanced, and cleavage at the second site now became clearly apparent (Figure 2), consistent with the possibility that phosphorylation of Scc1 by Plk1 increases the cleavability of Scc1. A cleavage product that corresponds to the fragment in between the two cleavage sites could not be detected under these conditions (Figure 2B), indicating that separase cleaves either one or the other site in one Scc1 molecule, but not or only rarely both.

It was suspected that the observed enhancement of Scc1's cleavability in the presence of Plk1 might be due to phosphorylation at two sites that are directly adjacent to the cleavage sites, Ser 175 and Ser 454, which had been found to be phosphorylated in mitosis in vivo (Figure 1C, Table 1). Therefore these two residues were mutated to alanine, thereby mutant Scc1-S175A/S454A was created (Figure 1C), and the cleavability of this mutant was tested in the absence or presence ofPlk1 in vitro. Scc1-S175A/S454A could still be cleaved at the first site, and this reaction was still slightly enhanced in the presence of P1k1, but cleavage at the second site was completely abolished even in the presence of Plkl (Figure 2B). These observations indicate that P1k1 enhances cleavage of Scc1 at Arg450 by phosphorylating Ser454.

### Figure 2:

(A,B) Human Scc1 was mutated at Ser175 (#5) and Ser454 (#7) to Alanine. Recombinant, ³⁵S-labeled wild type and mutant Scc1 tagged with 9xmyc at the C-terminus, were incubated with human separase. Recombinant human GST-Plk1 was added to the reaction mixtures where indicated. Samples where withdrawn from the reactions at the indicated timepoints, and analyzed by SDS-PAGE followed by immunoblotting (A) and Phosphorimager analysis (B). Arrows indicate full length Scc1-myc (fl Scc1-myc), C- and N-terminal fragment resulting from cleavage at Arg172 (Ct of 1st, Nt of 1st), and C- and N-terminal fragment resulting from cleavage at Arg450 (Ct of 2nd, Nt of 2nd). The upper part of the membrane or gel (fl Scc1-myc and Ct of 1st) is exposed for shorter than the lower part. Note that the enhancement of cleavage at Arg172 by Plk1 can be seen by comparing the intensities of the N-terminal fragments (Nt of 1st).

### Example 3

### Scc1 Phosphorylation is not Essential for the Dissociation of Cohesin from Chromosome Arms and for Progression through Mitosis

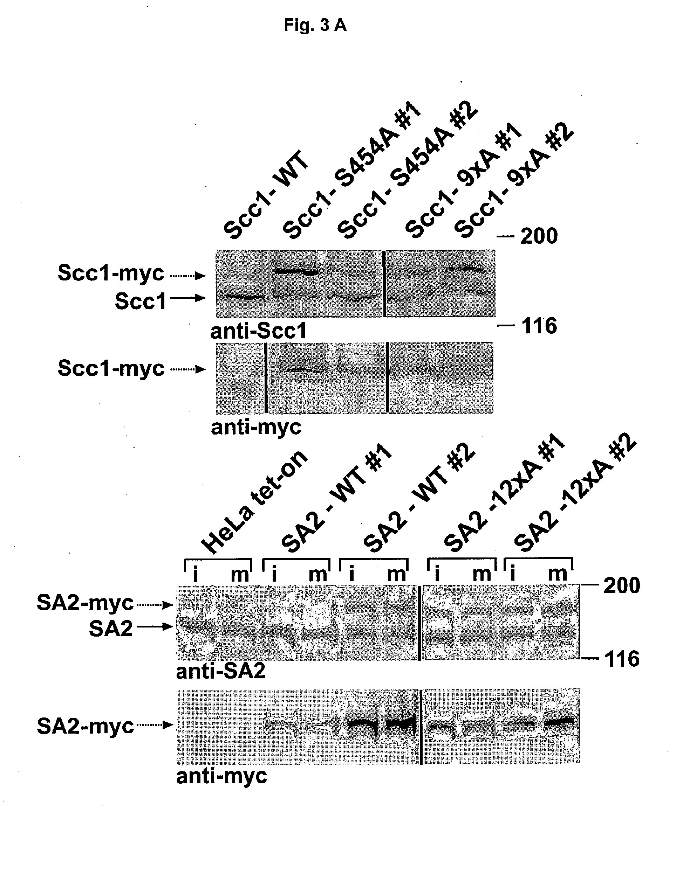

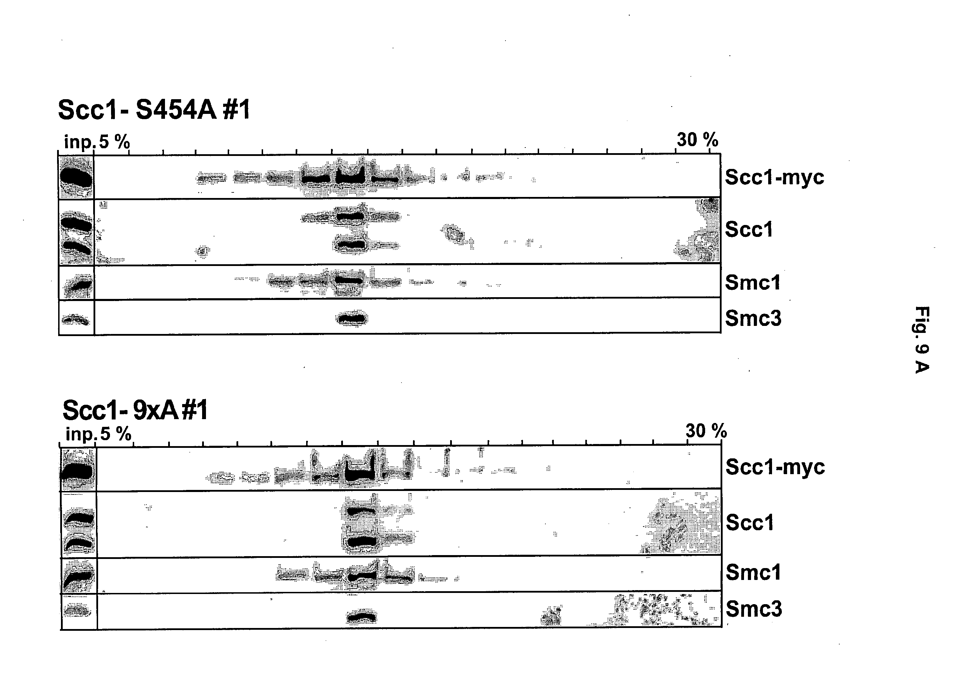

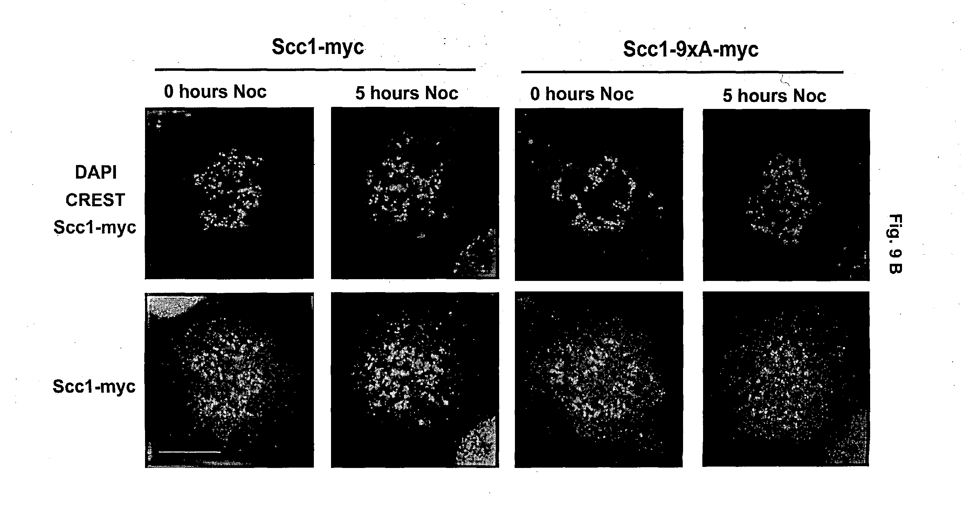

To address the physiological significance of Scc1 phosphorylation and the resulting enhanced cleavability of Scc1 cell lines were generated that stably express non-phosphorylatable versions of Scc1. The inventors either substituted only Ser454, or simultaneously 9 out of the 10 identified serine and threonine residues that were phosphorylated in vivo, by alanine residues, thereby generating mutants Scc1-S454A and Scc1-9xA, respectively (Figure 1C; a Scc1-10xA mutant could not be made for technical reasons). To be able to distinguish the ectopically expressed Scc1 proteins from endogenous Scc1 the Scc1 mutants were tagged with 9 mycepitopes at their C-termini. It has previously been shown that such a tag does not detectably compromise the ability of Scc1 to assemble into cohesin complexes (Waizenegger et al., 2000) that can establish cohesion (Hauf et al., 2001). Since expression of mutant Scc1 could have deleterious effects on cells, the inventors furthermore used a tetracycline-regulatable promoter to be able to regulate the expression of ectopic Scc1. To avoid potential overexpression artefacts the inventors screened by immunoblotting for cell lines in which the ectopically expressed Scc1 is present in amounts that are similar to the amounts of endogenous Scc1 (Figure 3A). Immunoprecipitation of the ectopically expressed Scc1 with myc antibodies, followed by SDS-PAGE and silver staining revealed that the mutated forms of Scc1 could associate with Smcl, Smc3 and SAl/2 into cohesin complexes (Figure 3B; note that endogenous untagged Scc1 does not co-immunoprecipitate with Sccl-myc, indicating that only one Scc1 molecule is present per cohesin complex). Sucrose density gradient centrifugation experiments in conjunction with immunoblotting showed that most of the ectopically expressed Scc1 was incorporated into cohesin complexes (Figure 9A). These observations indicate that any phenotypes (but also the absence of phenotypes) that are observed after ectopic expression of Scc1 are not simply caused by overexpression or by the inability of Scc1 to assemble into cohesin complexes.

Immunofluorescence analysis demonstrated that the localization of Scc1-S454A and Scc1-9xA was very similar to the one of wild type Scc1 (Figure 9B). Both mutants were present in nuclei from telophase throughout interphase until the next mitosis. In prometaphase, the bulk of mutant cohesin complexes had dissociated from chromosome arms, but small amounts remained at centromeres, even if prometaphase was prolonged by treatment of the cells with nocodazole (Figure 9B). Scc1 phosphorylation is therefore neither essential to load cohesin onto chromatin, nor to remove cohesin from chromosome arms in early mitosis. Furthermore, no obvious abnormalities were observed at later stages of mitosis and in the overall ability of cells expressing non-phosphorylatable Scc1 to proliferate, indicating that Scc1 phosphorylation is also not essential for the ability of separase to cleave cohesin complexes and to initiate anaphase. The finding that cells expressing Scc1 mutants in which Ser454 cannot be phosphorylated and in which cleavage at Arg450 is therefore compromised (Figure 2B) do not show anaphase defects is in agreement with our previous observation that Scc1 cleavage at Arg172 is sufficient for the viability of HeLa cells ( Hauf et al. 2001).

### Figure 3:

(A) Wild-type Scc1 or SA2, or the indicated mutant proteins (also see Figure 1C), all tagged with 9xmyc at the C-terminus, were stably and inducibly expressed in HeLa tet-on cells. After induction by treatment with 2 µg/ml Doxycycline for 1 to 3 days, cell extracts were prepared from either logarithmically growing cells (left panel; 'i' (interphase) in the right panel) or from cells arrested in mitosis by Nocodazole ('m' (mitosis) in the right panel). Exogenous protein was detected by immunoblotting with anti-myc antibodies (lower panel). Since the 9xmyc-tag caused a reduced mobility in SDS-PAGE compared to the endogenous protein, Scc1- and SA2-immunoblots revealed the relative amounts of exogenous and endogenous protein in the different cell lines (upper panel).
(B) Extracts were prepared from the different cell lines as indicated, and immunoprecipitation was performed using myc-antibodies, followed by SDS-PAGE and Silver staining. As a control, cohesin complex was immunoprecipitated from untransfected HeLa tet-on cells using antibodies to SA2.
(C) Extracts were prepared from SA2 WT-myc or SA2 12xA-myc expressing cells, and fractionated by sucrose density gradient centrifugation, followed by immunoblotting with antibodies recognizing the proteins indicated on the right. (inp. = unfractionated sample of the extract).

### Figure 9:

(A) Extracts were prepared from Scc1 S454A-myc or Scc1 9xA-myc expressing cells, and fractionated by sucrose density gradient centrifugation, followed by immunoblotting with antibodies recognizing the proteins indicated on the right. (inp. = unfractionated sample of the extract).
(B) Scc1 WT-myc or Scc1 9xA-myc expressing HeLa cells were either grown logarithmically (0 hours noc) or arrested in prometaphase for 5 hours by Nocodazole (5 hours noc). Cells were extracted prior to fixation, and stained with myc-antibodies. Kinetochores were labeled with CREST serum, and DNA was counterstained with DAPI.

### Example 4

Phosphorylation of SA2 is Essential for the Dissociation of Cohesin from Chromosomes during Prophase and Prometaphase

The observation that non-phosphorylatabale Scc1 mutants bind chromatin in interphase and dissociate from chromosome arms normally in early mitosis implied that the requirement for Plk1 in cohesin dissociation and the inhibitory effect of cohesin phosphorylation on chromatin binding (Sumara et al., 2002; Giménez-Abian et al., 2004) cannot be explained by Scc1 phosphorylation. It was therefore asked whether phosphorylation of SA2 might control the association of cohesin with chromosomes. First a mutant of SA2 was generated in which 12 serine and threonine residues that were phosphorylated in mitosis in vivo were mutated to alanine residues, called SA2-12XA (Figure 1-C). This mutant and wild type SA2 were C-terminally tagged with myc epitopes and expressed both in HeLa cells in a stable and inducible manner, employing the same strategy that had been used for Scc1. Also in this case cell lines were isolated in which the levels of ectopically expressed SA2 are similar to the levels of endogenous SA2 (Figure 3A). Immunoprecipitation experiments with myc antibodies and sucrose density gradient centrifugation showed that both the tagged SA2 wild type and SA2-12xA protein were incorporated into cohesin complexes (Figure 3B and C).

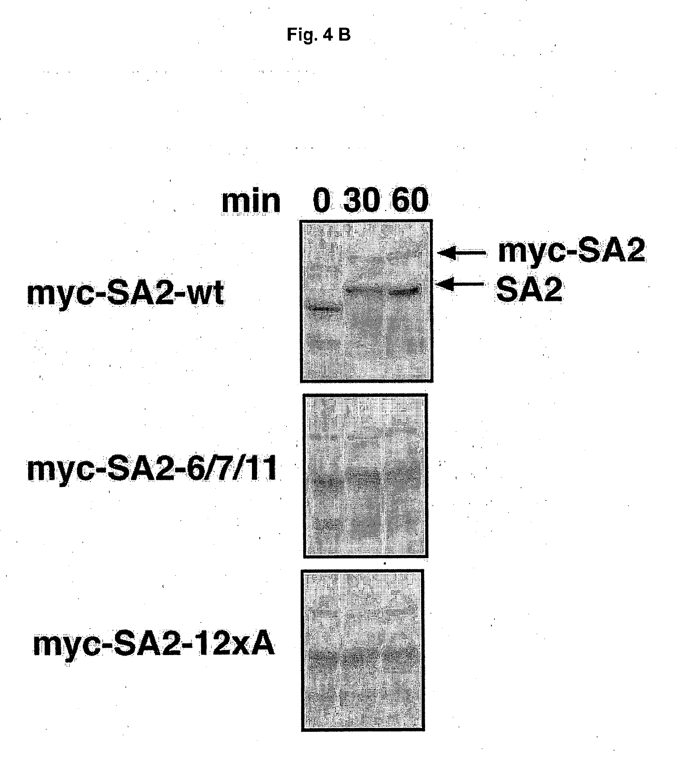

Since SA2 phosphorylation can be catalyzed by purified Plk1 in vitro and depends on Plx1 in Xenopus egg extracts (Sumara et al., 2002) it was asked if SA2-12xA had lost the ability to be phosphorylated by Plk1. When purified cohesin complexes were incubated with Plk1 and ³²P-γ-ATP, approximately 50 % less radiolabel was incorporated into SA2-12xA than into SA2 wild type, whereas phosphorylation of Scc1 in the same complexes was not affected (Figure 4A). Because both budding yeast and human Polo-like kinases can phosphorylate many sites in vitro that are not phosphorylated in vivo (Shou et al., 2002; Kraft et al., 2003) it is possible that the residual phosphorylation of SA2-12xA by Plk1 in vitro occurs on sites that are not phosphorylated in vivo. Therefore the phosphorylation of wild type and mutant forms of SA2 was analyzed under more physiological conditions by incubating ³⁵S-labeled recombinant SA2 in mitotic Xenopus egg extracts. SA2 phosphorylation in these extracts depends on Plx1 (Sumara et al., 2002) and causes an electrophoretic mobility shift (Figure 4B). This shift was partially abolished when three C-terminal phosphorylation sites were mutated (mutant SA2-6/7/11) and no shift could be seen with SA2-12xA (Figure 4B). These results indicate that most mitosis-specific phosphorylation sites have been removed from SA2-12xA. The finding that mitosis-specific phosphorylation sites in SA2 are clustered in the C-terminus was also confirmed by generating deletion mutants in which SA2 was progressively shortened from the C-terminus, because SA2's mobility shift was completely abolished when at least 124 C-terminal residues were removed (Figure 4C).

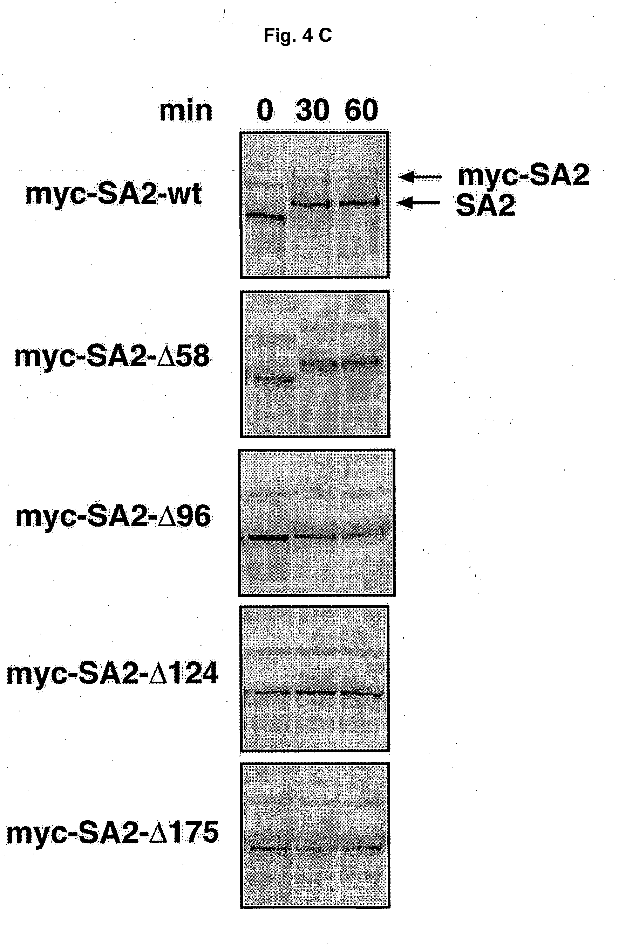

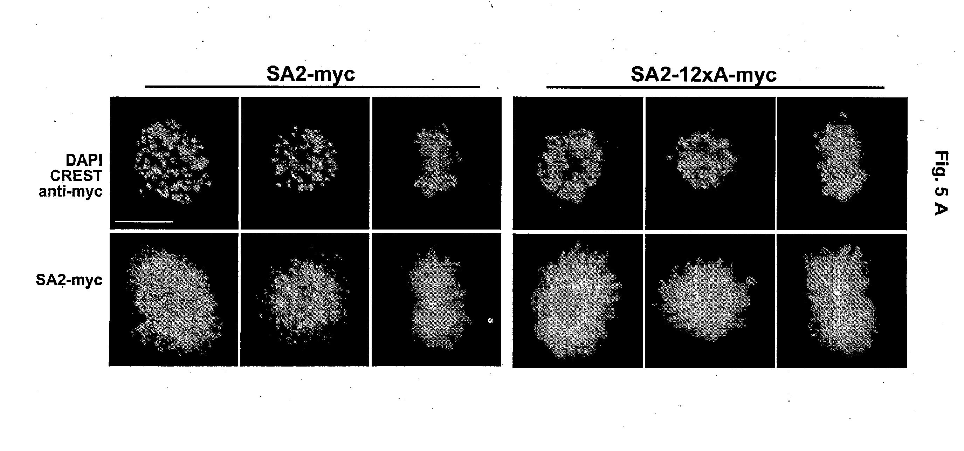

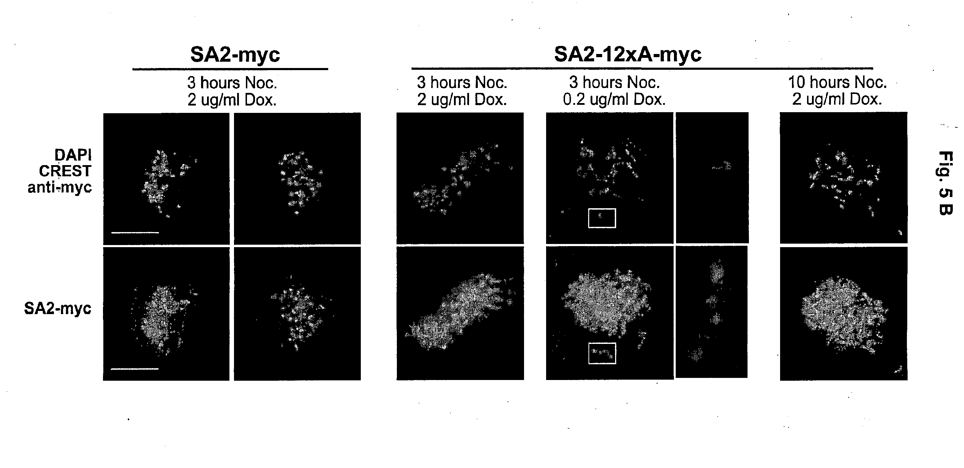

To be able to address the physiological relevance of SA2 phosphorylation it had to be analyzed first if cohesin- complexes containing tagged SA2 behave normally in human cells. Immunofluorescence imaging of wild type SA2-myc showed a localization pattern that is very similar to the pattern found for Scc1 (Waizenegger et al., 2000; Hauf et al., 2001; Giménez-Abian et al., 2004). SA2-myc was nuclear in interphase, and mainly present in a soluble form in the cytoplasm during mitosis. When mitotic cells were extracted, to remove the soluble pool, a minor fraction bound to chromatin was found, and in prometaphase and metaphase SA2-myc was enriched at centromeres as compared to chromosome arms (Figure 5A). The tag in SA2 does therefore not seem to interfere with the behavior of cohesin complexes containing SA2-myc.

When the intracellular distribution of the SA2-12xA mutant was analyzed by immunofluorescence microscopy, it was found that this protein was also nuclear throughout interphase and that the nuclear signal could only partially be reduced by removing soluble cohesin complexes by pre-extraction, indicating that complexes containing SA2-12xA can associate with chromatin like wild type cohesin. In stark contrast to wild type SA2, however, SA2-12xA was not strongly enriched at centromeres of prometaphase and metaphase chromosomes but instead was almost equally abundant on chromosome arms and on centromeres (Figure 5A). A very similar distribution of cohesin on chromosome arms and centromeres has been observed after depletion of Plk1 by RNAi (Giménez-Abian et al., 2004).

These observations indicated that phosphorylation of SA2 is not required for the loading of cohesin onto chromatin, but is essential for its dissociation from chromosome arms during early mitosis. However, it also remained possible that cohesin complexes containing SA2-12xA simply dissociated from chromosomes more slowly than wild type complexes. To address this possibility cells were analyzed in which prometaphase was prolonged by treatment with nocodazole. Under these conditions, complexes containing wild type SA2 dissociated completely within three hours from chromosome arms but remained at centromeres (Figure 5B), confirming earlier observations made for Scc1 (Waizenegger et al., 2000; Hauf et al., 2001; Giménez-Abian et al., 2004). In contrast, SA2-12xA could still clearly be detected on chromosome arms after threee hours of nocodazole treatment and even after 10 hours (Figure 5B), excluding the possibility that the dissociation of cohesin complexes containing this mutant is simply slower than the dissociation of wild type complexes.

### Figure 4:

(A) Wild-type SA2 or SA2 12xA containing cohesin complexes were immunopurified by anti-myc antibodies from the respective cell lines. Similar amounts of cohesin were incubated with recombinant GST-Plk1, and the amount of phosphorylation was quantified by ³²P incorporation followed by Phosphorimager analysis and quantification using ImageJ.
(B,C) In vitro translated ³⁵S-labeled SA2 tagged at the N-terminus with 9xmyc was incubated in interphase Xenopus egg extracts, which were induced to enter mitosis at timepoint 0 min by addition of non-degradable cyclin B Δ90 and ocadaic acid (wt = wild type, (B): phosophorylation site mutants, (C): C-terminal deletion mutants). Samples were collected at the indicated timepoints and analyzed by SDS-PAGE followed by Phosphorimager analysis. The slower migrating band presumably represents myc-SA2 whereas the faster migrating band is generated by translation initiation at an internal start codon.

### Example 5

### Cohesin Complexes Containing Non-Phosphorylatable SA2 are Able to Establish Cohesion

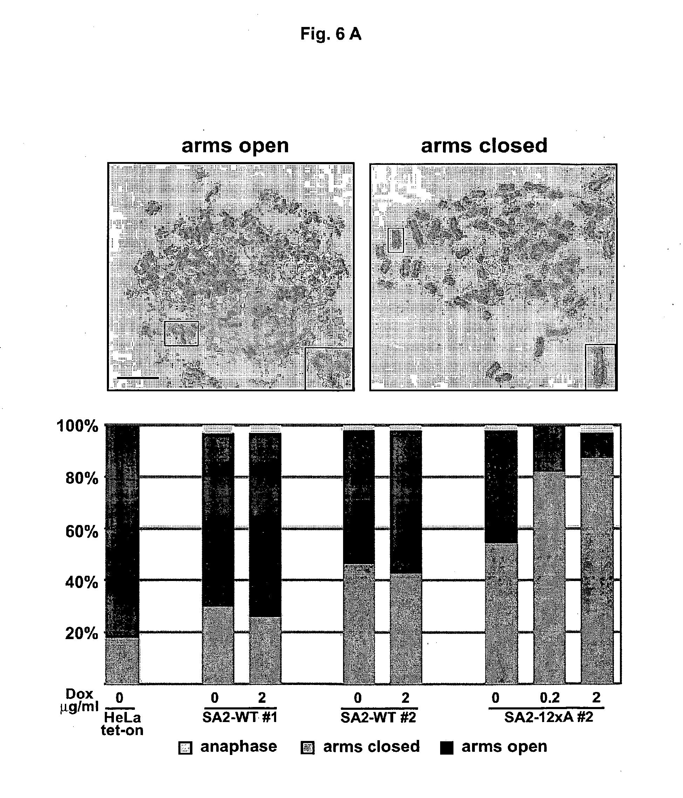

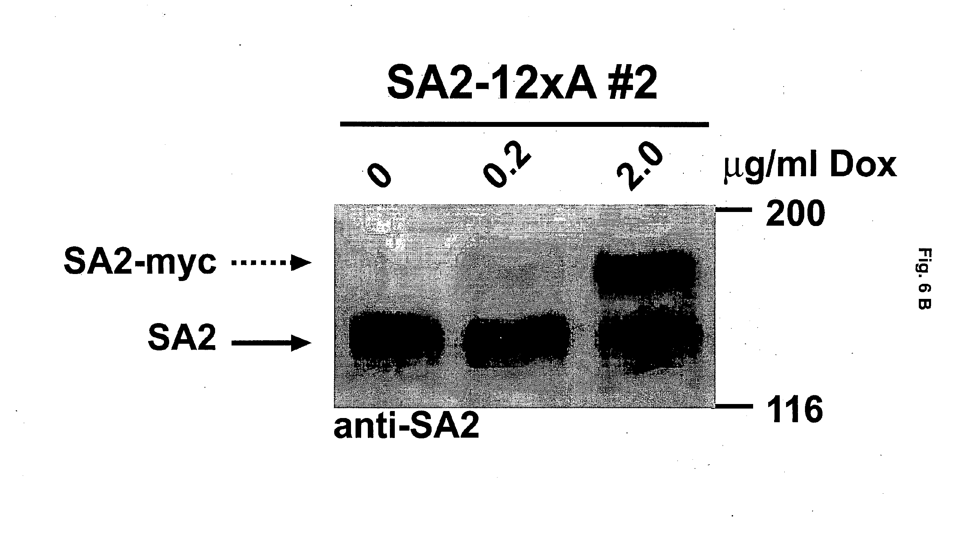

It was also possible that the amino acid exchanges in SA2-12xA had rendered the protein non-functional, possibly resulting in the formation of complexes that bound chromatin non-specifically and therefore did not dissociate from chromosomes in mitosis. To test this possibility it was asked if cohesin complexes containing SA2-12xA are able to establish sister chromatid cohesion. To achieve this, cells were treated again with nocodazole, which normally results in the loss of cohesion between chromosome arms but not at centromeres, resulting in the formation of "X-shaped" chromosomes with "open arms" that can be seen by chromosome spreading and Giemsa staining (Giménez-Abian et al., 2004). In this assay, chromosomes from most cells expressing wild type SA2-myc showed the normal "open arm" phenotype (between 55 and 71 %, depending on the cell line analyzed; Figure 6A). In contrast, only 10 % of mitotic cells expressing SA2-12xA contained chromosomes whose arms had lost cohesion during the nocodazole arrest, whereas 87 % had maintained cohesion between chromosome arms (Figure 6A). This result strongly indicated that the cohesin complexes that contain SA2-12xA and that are remaining on chromosome arms in prometaphase (Figure 5B) are able to establish and maintain cohesion between sister chromatids.

Finally, to further confirm this hypothesis, it was asked if the ability of SA2-12xA expressing cells to maintain arm cohesion during a nocodazole treatment depends on the amount of ectopic protein that is expressed. Therefore cells containing SA2 12xA transgenes were treated with different doses of doxycycline and the levels of exogenous SA2 with the arm cohesion phenotype were compared. The levels of mutant SA2-myc could be well controlled by the amount of doxycyclin used (Figure 6B), and there was a clear correlation between the amount of SA2-12xA and the number of cells whose chromosomes maintained arm cohesion during treatment with nocodazole, whereas expression of wild type SA2-myc had no significant influence on arm cohesion. (Figure 6A). Cells of the SA2-12xA cell line maintained arm cohesion slightly more frequently than control cells even if SA2-12xA expression was not induced, but it is possible that small amounts of the ectopic protein are also synthesized in the absence of doxycycline.

These observations rule out the possibility that the different frequencies with which arm cohesion is observed after nocodazole treatment in SA2-12xA and control cells are due to other differences in the cell lines than expression of the different transgenes. It was therefore concluded that cohesin complexes containing SA2-l2xA are able to maintain and establish cohesion, but that these complexes are unable to dissociate from chromosomes. Phosphorylation of SA2 at its C-terminus therefore appears to be essential for the unloading of cohesin from chromosome arms during prophase and prometaphase.

### Figure 5:

(A) Logarithmically growing SA2 WT-myc or SA2 12xA-myc expressing HeLa cells were extracted prior to fixation, and stained with myc-antibodies. Kinetochores were labeled with CREST serum, DNA was counterstained with DAPI.
(B) SA2-myc expression was induced by different amounts of Doxycycline, and cells were arrested in prometaphase by Nocodazole treatment for 3 or 10 hours. Cells were spun on glass slides, extracted by detergent, fixed and processed for immunostaining as in (A).

### Example 6

### Phosphorylation of SA2 is Required for Efficient Resolution of Sister Chromatids

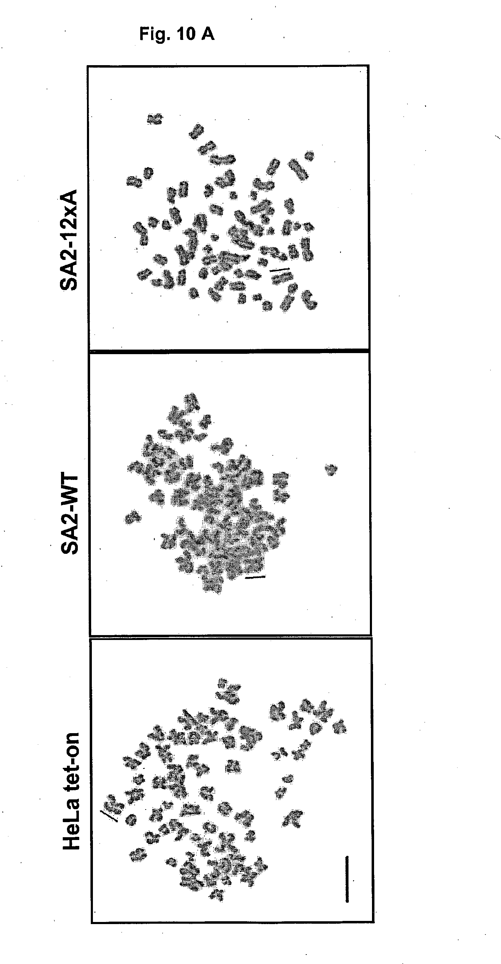

The purpose of these experiments was to address the question how chromosome structure is influenced by expression of non-phosphorylatable SA2. It was found that neither condensin binding nor the compaction nor the shortening of chromosomes in a prolonged mitotic arrest was detectably influenced by expression of SA2-12xA (Figure 10), further strengthening the notion that condensin binding does not require cohesin dissociation.

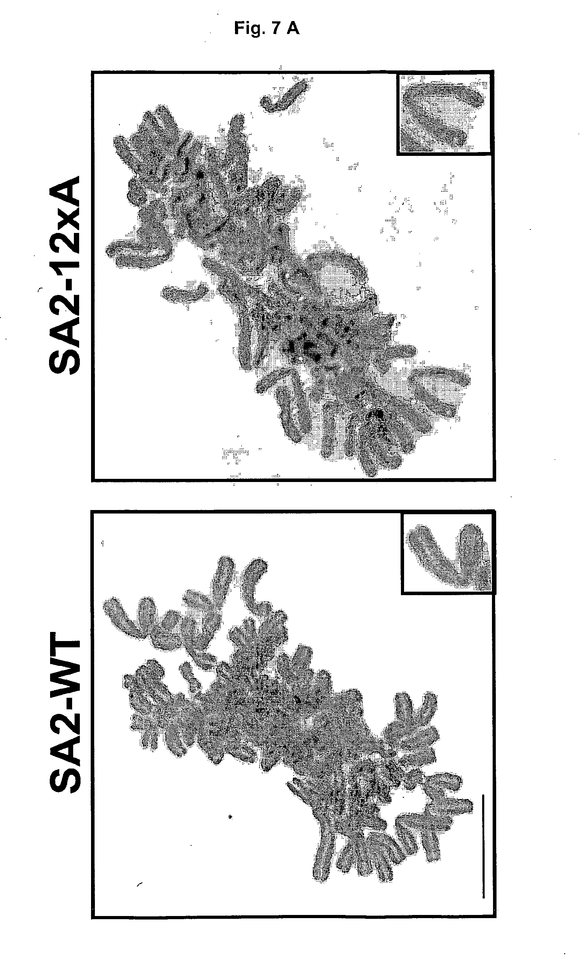

In contrast, it was noticed that sister chromatid arms often stayed in closer proximity in SA2-12xA-expressing cells compared to controls (Figure 7A). The interchromatid distance during prometaphase and metaphase was measured and within the same cell line a variation from around 0.4 to 1.1 um between individual cells was found (Figure 7B). This variability is presumably caused by different periods of time that cells have spent in mitosis, i.e. cells in which the inter-chromatid distance was small might have spent shorter time in mitosis than the ones where sister chromatids were more resolved. It was also found that there was some variability between different cell lines. However, cells expressing wild type SA2 showed no difference in the average inter-chromatid distance in the absence or presence of exogenous SA2, whereas the average inter-chromatid distance was progressively shortened when SA2 12xA-myc was expressed in increasing amounts (Figure 7B).

Since resolution of sister chromatid arms happens progressively during pro- and prometaphase, a reduction in the average distance between sister chromatids might also be caused by shortening the time up to metaphase (therefore leaving cells less time to resolve sisters). However, when the percentage of different mitotic stages in SA2-12xA versus wild type SA2 expressing cells was compared, no indication for a shortening of prometaphase was found. Phosphorylation of SA2 and dissociation of cohesin from chromosome arms therefore appear to be required for the efficient resolution of sister chromatid arms.

### Figure 6:

(A) Cells were cultured in the absence or presence of different amounts of Doxycycline as indicated. After arrest in Nocodazole for 3 hours, cells were fixed, spread on glass slides, and stained with Giemsa. The number of cells with chromosome arms that had opened or that were connected was scored as indicated.
(B) Whole cell extracts were prepared from HeLa cells expressing SA2 12xA-myc after treatment with increasing amounts of Doxycycline (0, 0.2, 2.0 µg/ml). The ratio of exogenous SA2 12xA-myc to endogenous SA2 was visualized by immunoblotting with antibodies to SA2.

### Figure 10:

(A) HeLa tet-on, wild-type SA2-myc or SA2 12xA-myc expressing HeLa cells were arrested in Nocodazole for 10 hours. Cells were fixed, spread on glass slides, and stained with Giemsa. The small bars next to one of the chromosomes in all panels have the same length.
(B) Wild-type SA2-myc or SA2 12xA-myc expressing HeLa cells were spread on glass slides, extracted prior to fixation, and immunostained as indicated.

### Example 7

### SA2 Phosphorylation is not Essential for Anaphase

The prolonged persistence of cohesin on chromosome arms and the closer proximity of sister chromatid arms might cause defects in anaphase, for example because larger amounts of cohesin cannot be cleaved efficiently enough, or because perturbance of chromosome structure might interfere with the separation of sister chromatids. However, no obvious anaphase defects in cells expressing non-phosphorylatable SA2 were observed. Furthermore, in all anaphases that were observed (n > 50), it was found that SA2-12xA had completely disappeared from separating chromatids (Figure 7C). This observation supports the notion that separase is not only able to cleave cohesin at centromeres but can also cleave cohesin on chromosome arms (Giménez-Abian et al., 2004) and indicates that separase can even cleave abnormally high amounts of cohesin on chromosomes. Scc1 cleavage products could be detected in cells expressing SA2-12xA, consistent with the interpretation that the loss of SA2-12xA containing cohesin complexes from chromatids in anaphase is mediated by separase. These observations indicate that SA2 phosphorylation is required for the dissociation of cohesin from chromosome arms during prophase and prometaphase, but that this dissociation process is not absolutely essential for the initiation of anaphase.

### Figure 7:

(A.B) HeLa cells expressing SA2 WT-myc or SA2 12xA-myc were treated with different amounts of Doxycycline as indicated. Cells were spread on glass slides, and chromosomes were stained with Giemsa. Representative cells from SA2 WT-myc or SA2 12xA-myc cell lines after induction with 2 µg/ml Doxycycline are shown in (A). More than 50 cells in prometa- or metaphase were picked from each sample, The distance between sister chromatids was determined for 5 chromosomes in each cell, and averaged. One dot in the diagram represents the average interchromatid distance from one cell. Squares indicate the average distance for all cells in a given sample.
(C) Representative immunofluorescence image of a normal anaphase in a cell expressing SA2 12xA-myc. The cell was not extracted prior to fixation, so that the soluble pool of SA2 12xA-myc is revealed by anti-myc staining.

### Example 8

### Generation of Phospho-Specific Antibodies to Cohesin Subunits

### a) Generation of antisera against phospho-epitopes on cohesin subunits

To generate antibodies that specifically react with mitotic phosphorylation sites on the cohesin subunits Scc1 and SA2, rabbits were immunized with synthetic phospho-peptides. These peptides represent those parts of the Scc1 or SA2 amino acid sequences which were found by mass spectrometry to be phosphorylated in human cultured cells specifically in mitosis (see Hauf et al., 2001; Table 1 and Figure 1). Two Scc1 and two SA2 peptides were synthesized with the following amino acid sequences (amino acid residues are listed in the one-letter code; pS and pT represent phospho-serine and phospho-threonine residues, respectively; the carboxy-terminal cysteine residue is added to allow subsequent coupling of the peptides to a carrier protein and is not derived from the Scc1 or SA2 sequences):
Peptide designated Scc1-1 (in the figures designated 895; corresponding to amino acid residues 449 to 458 of human Sccl; SEQ ID NO: 1).
Peptide designated Scc1-2 (in the figures designated 896; corresponding to amino acid residues 40 to 51 of human Sccl; SEQ ID NO:1).
Peptide designated SA2-1 1 (in the figures designated 897; corresponding to amino acid residues 1113 to 1122 of human SA2; SEQ ID NO:2).
Peptide designated SA2-2 (in the figures designated 898; corresponding to amino acid residues 1147 to 1157 of human SA2; SEQ ID NO:2).

The synthetic peptides were coupled via the carboxy-terminal cysteine residue to keyhole limpet hemocyanin (KLH; Merck Biosciences, Nottingham, UK) following the manufacturer's instructions. 3 mg of lyophilized peptide were dissolved in 700 µl of buffer containing 3 M guanidine hydrochloride and 0.5 M sodium tetraborate, pH 6.9, and the solution is added to 300 µl of the same buffer in which 3 mg KLH has been dissolved. After removal of oxygen by aerating with argon, the reaction mixture is incubated for 1 hour at room temperature and subsequently stored at 4°C. To determine the efficiency of the coupling reaction, 10 µl aliquots of the peptide-KLH solution were removed at the beginning and the end of the reaction, mixed with 50 µl of Ellman's reagent (50 mM sodium acetate, 2 mM 5,5'-Dithiobis(2-nitrobenzoic acid); Sigma-Aldrich, St.Louis, MO), 100 µl solution containing 1 M Tris-HCl, pH 8.0 and 840 µl water, and the absorbance of the samples is measured at 412-nm ("Ellman test"). A decrease in absorbance indicates a reduction in the amount of free thiol groups and is a measure for the efficiency of peptide coupling to KLH (Ellman, 1959; Bulaj et al., 1998). The coupled peptides were used to immunize rabbits at a commercial facility (Gramsch Laboratories, Schwabhausen, Germany). The resulting sera were frozen in 20 ml aliquots in liquid nitrogen and stored at -80 °C.

### b) Generation of peptide affinity columns for the purification of phospho-specific antibodies to cohesin subunits

To purify phospho-specific antibodies from the rabbit sera, peptide affinity columns were generated. Each of the cohesin phospho-peptides described above is coupled to a chromatography resin, and the resulting columns were then used to bind those antibodies that specifically react with these phospho-peptides. In addition, peptides were synthesized that are identical in their sequences to the sequences of the phospho-peptides, except that they contain unmodified serine and threonine residues instead of the phospho-serine and phospho-threonine residues, respectively. These peptides were also coupled to chromatography resins, and the resulting columns were used to remove antibodies from the rabbit sera that react with the cohesin peptides independently of their phosphorylation state.

To generate the peptide affinity columns, Poros beads (Applied Biosystems, Foster City, CA) were activated with the bi-functional cross-linker sulfo-GMBS (Pierce, Rockford, IL) according to the manufacturer's instructions, which results in the generation of maleimid groups. For each of the synthetic peptides, 5 mg of lyophilized peptide were dissolved in 3 ml buffer containing 3 M guanidine hydrochloride and 0.1 M Hepes-KOH, pH 7.9, yielding a peptide concentration of ~2.27 µmol. 310 mg of activated Poros beads were mixed with the peptide solution and the suspension is incubated on a rotary shaker for one hour at room temperature. Subsequently, the Poros beads were collected by low speed centrifugation and the coupling reaction is stopped by washing with at least 10 bead volumes of a buffer containing 150 mM NaCl, 20 mM Tris-HCl, pH 7.5. The efficiency of cross-linking is determined by the Ellman test (see 1.). Resin coupled to peptides were stored in batch at 4 °C. To obtain homogenously packed affinity columns the coupled resin is transferred into a HPLC column (0.7 ml; Applied Biosystems) by employing a filling device (Applied Biosystems) that is connected to a BIOCAD HPLC system (Applied Biosystems):

### c) Purification and concentration of phospho-specific antibodies to cohesin subunits

Phospho-specific antibodies were purified from the sera generated as described under a) by the following strategy: Antibodies that react with cohesin peptides independently of their phosphorylation state were first removed from each serum by passing it over the affinity column that contains the corresponding antigenic peptide in its non-phosphorylated form. Subsequently the serum is passed over a second affinity column which contains the phosphorylated antigenic peptide. Both columns were connected in line to a BIOCAD HPLC and were equilibrated with 20 column volumes (cv) of HBS buffer (20 mM Hepes-KOH, 150 mM NaCl) at a flow rate of 7 ml/min. All chromatography steps were carried out at room temperature and were monitored by measuring the absorbance on-line at 280 nm. Aliquots of antiserum were rapidly thawed in a water bath at 37°C. The thawed serum is extracted once for two minutes with an equal volume of chloroform to remove lipids. To separate the organic fraction from the serum, the emulsion is centrifuged at 2, 000 rpm in a Varifuge 3.0R (Heraeus; Kendro, Asheville, NC) for 15 minutes at 4 °C. Subsequently, the serum is filtered through a 0.45 µm syringe filter (MILLEX-HA; MILLIPORE, Billerica, MA) and stored on ice. Antibodies were purified from 20 ml of serum by four subsequent chromatography runs in each of which 5 ml aliquots were processed. 5 ml of chloroform extracted serum were loaded onto the column with a flow rate of 5 ml/min (column pressure: 20-25 bar). The column is washed with 50 cv of HBS.

Subsequently the two columns were disconnected and bound proteins were eluted separately from the two columns. Bound proteins were eluted first with Buffer A (1.5 M MgCl₂, 100 mM sodium acetate pH 5.2) and subsequently by Buffer B (100 mM glycine-HCl, pH 2.45). The pH of the eluted fractions is immediately neutralized by the addition of 1/10 volume of 2 M Hepes-KOH, pH 7.9. The fractions obtained by elution with Buffer A or with Buffer B were kept separate during all subsequent steps because antibodies eluted by high MgCl₂ concentrations (Buffer A) or by low pH (Buffer B) can differ in their abilities to recognize their antigen under different conditions. The peak fractions were dialyzed over-night at 4 °C against 2 liters of buffer C (20 mM Hepes-KOH, 150 mM NaCl, 10% glycerol, 0.5 mM DTT), using dialysis tubing with a molecular weight cut-off (MWCO) of 3, 500 Da (Spectra/Por; Spectrum Laboratories, Rancho Dominguez, CA). Buffer C is exchanged once after 3 hours. Subsequently, the antibodies were concentrated by ultra-filtration in a Centriprep centrifugal filter device (YM-30, MWCO 30 kDA, 15 ml, Amicon; MILLIPORE) at 3, 000 rpm (Varifuge 3.OR) and 4°C. The final protein concentration is determined by photometric measurements at a wavelength of 280 nm. An OD₂₈₀ = 1 of a mixture of IgG and IgM corresponds to 1.4 mg/ml protein (Harlow and Lane, 1988) purity of the samples is assessed by SDS-PAGE and silver staining, and 500 µl aliquots of the purified antibodies were frozen in liquid nitrogen and stored at -80°C. A typical purification yields 6 to 9 mg of pure antibodies from 20 ml of antiserum.

### d) Characterization of the specificity of phospho-cohesin antibodies

The following experiments were performed to determine if the antibodies that were eluted from the affinity columns containing phosphorylated antigenic peptides are specifically reacting with phosphorylated cohesin.

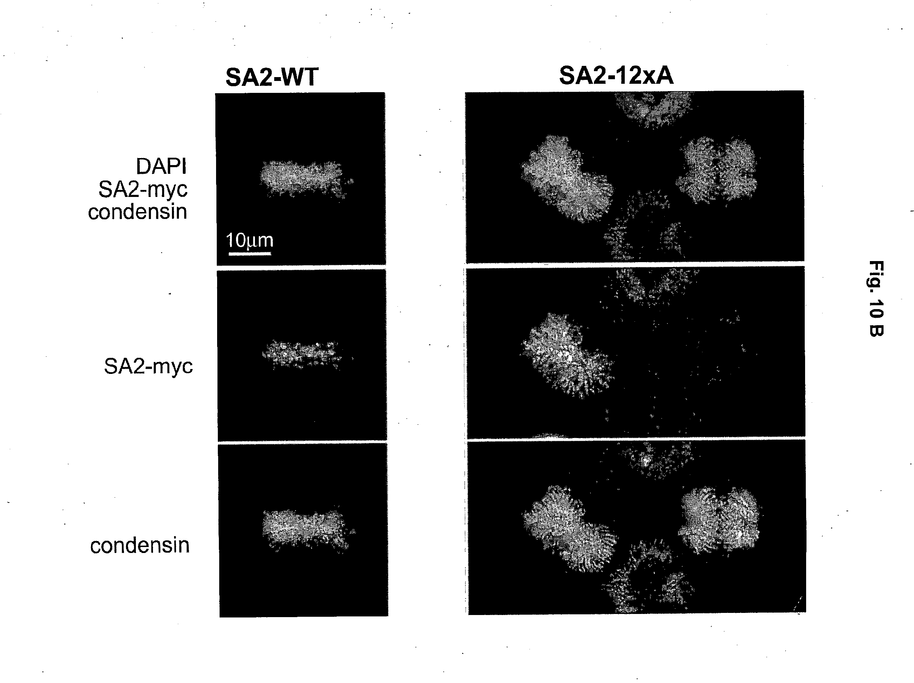

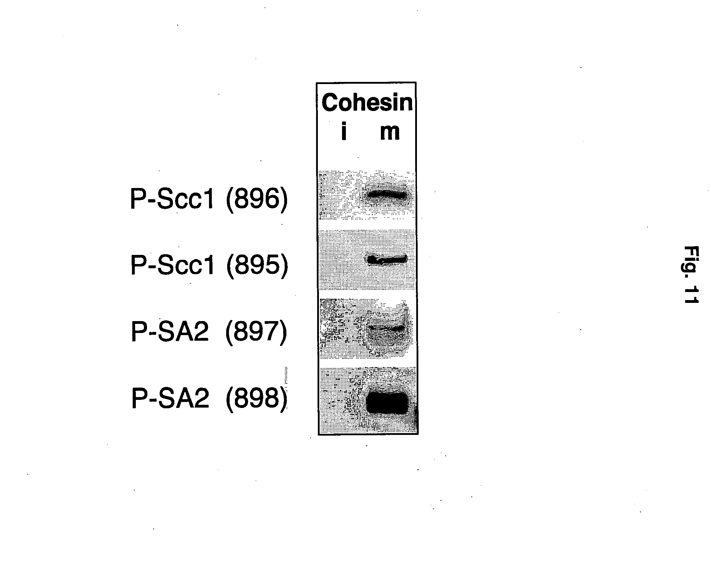

First, cohesin is isolated by immunoprecipitation with cohesin specific antibodies from cultured human HeLa cells that are arrested either in interphase by treatment with hydroxyurea or in mitosis by treatment with nocodazole (see Figure 1 in Hauf et al., 2001). Proteins in the immunoprecipitates were separated by SDS-PAGE and analyzed by immunoblotting with the antibodies that were raised against the cohesin phospho-peptides number Scc1-1, Scc1-2, SA2-1 and SA2-2 . For this purpose, proteins separated by gel electrophoresis were transferred to PVDF blotting membranes and incubated with the antibodies purifed as described under 3. The reactivity of the antibodies with proteins bound to the PDVF membrane is detected by incubation with enzyme-linked secondary antibodies that can be detected by enhanced chemoluminescence (ECL). As is shown in Figure 11, the antibodies that have been generated against the phospho-peptides Scc 1 - 1, Scc 1 -2, SA2-1 and SA2-2 only react with cohesin subunits that have been isolated from mitotic cells where the cohesin subunits Scc1 and SA2 are phosphorylated, whereas the antibodies do not react with cohesin subunits from interphase cells where Scc1 and SA2 are not phosphorylated (see Figure 1 of Hauf et al., 2001). These results are consistent with the notion that the reactivity of the antibodies with cohesin subunits depends on the mitosis specific phosphorylation of these subunits.

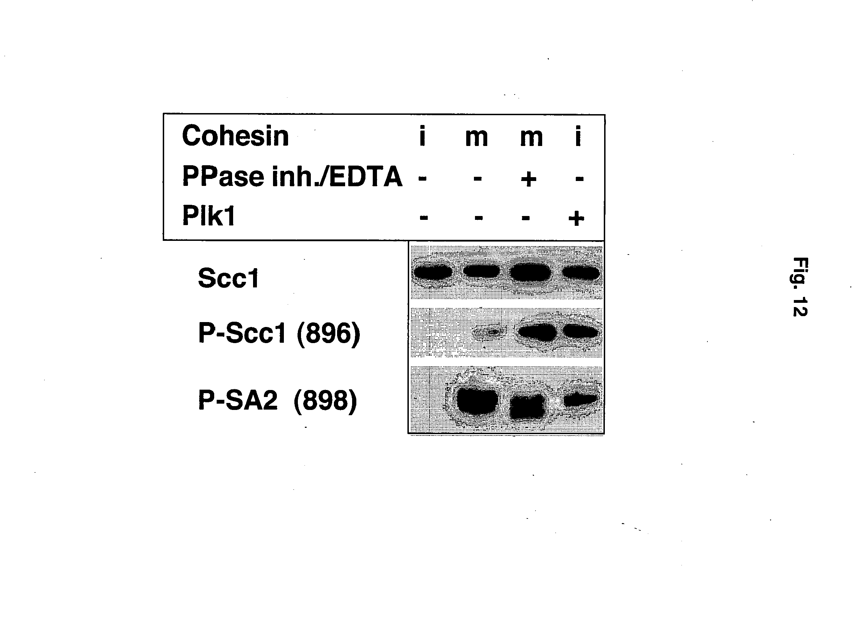

Second, to test the hypothesis that the reactivity of the antibodies with cohesin depends on cohesin phosphorylation by Plk1 the following experiments were performed. Immunoprecipitated interphase cohesin is incubated in buffer containing MgCl₂ and ATP in either the absence of presence of recombinant active Plk1 enzyme. Subsequently, the cohesin subunits were analyzed by SDS-PAGE and immunoblotting with the phospho-antibodies as described above. As is shown in Figure 12 for antibodies raised against the phospho-peptides 896 (Scc1-2) and 897 (SA2-1), the pre-treatment of interphase cohesin with MgCl₂, ATP and Plkl allows the antibodies to react with cohesin, whereas interphase cohesin pre-incubated in MgCl₂ and ATP in the absence of Plkl is not recognized. These data imply that the reactivity of the antibodies with cohesin depends on the prior phosphorylation of cohesin which can be catalyzed by Plk1 in vitro.

Figure 12 also shows that antibodies raised against phospho-peptide 896 (Scc1-2) react better with the cohesin subunit Scc1 if cohesin is isolated from cells in the presence of chemical inhibitors of protein phosphatases (PPase inh.). To exclude the possibility that the addition of phosphatase inhibitors to cell lysates allows protein kinases to generate phospho-sites on cohesin that are not normally present in vivo, EDTA is added which chelates MgCl₂ and thereby prevents protein kinases from utilizing ATP. The data in Figure 12 suggest that the 896 (Scc1-2) antibodies recognize a phospho-epitope on Scc1 which is partly removed by protein phosphatases under normal isolation conditions and which can therefore be protected by the addition of phosphatase inhibitors. The data therefore further support the notion that the 986 antibodies are phospho-specific,

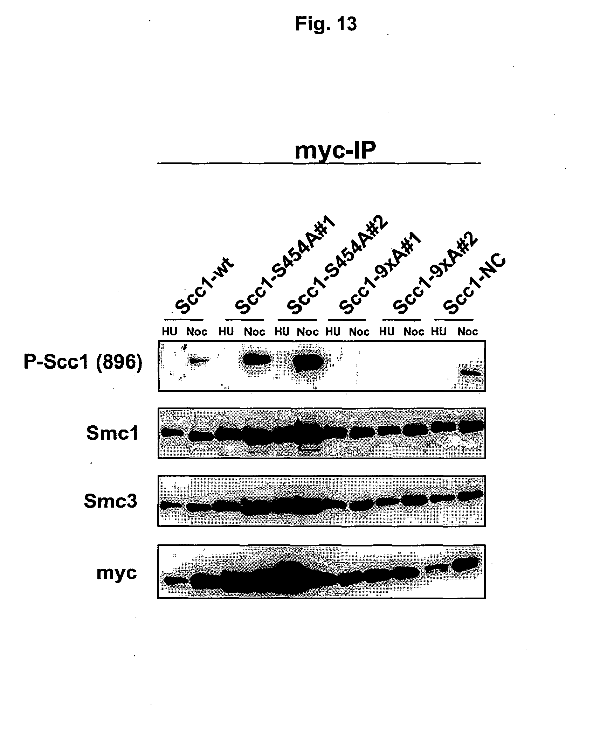

Third, to directly test if the reactivity of the antibodies with cohesin subunits depends on phosphorylation of the amino acid residue against which the antibodies were raised the following experiments were performed: Point mutations were introduced in the cohesin cDNAs which result in mutation of those amino acid residues that are normally phosphorylated in mitosis and against which the antibodies were raised. Specifically, mutations were chosen that change the codons fom serine or threonine residues into codons for alanine residues which can not be phosphorylated. The mutated cDNAs were fused to a cDNA fragment that encodes multiple copies of the myc epitope to facilitate subsequent detection of the mutated protein when expressed in cultured cells (see Figure 1C in Hauf et al.). As is described above (Figure 3, Hauf et al.) the resulting mutants were expressed stably in cultured human HeLa cells. Cohesin complexes that contain the mutated cohesin subunit were isolated by immunoprecipitation using antibodis specific for the myc epitope and were analyzed by SDS-PAGE and immunoblotting with the phospho-antibodies. As is shown in Figure 13 for the 896 (Scc1-2) antibodies, the reactivity of the phospho-antibodies with Scc1 is abolished by mutation of the phosphorylation site against which the antibodies were raised. The 896 (Scc1-2) antibodies were raised against a peptide that contains serine 46 in a phosphorylated form. Correspondingly, these antibodies can react with those forms of Scc1 in which serine 46 has not been mutated (Scc1 wt, wild type Scc1; Scc1-S454A, a form ofScc1 in which serine 454 has been mutated to alanine; Scc 1 -NC, a form of Scc1 that can not be cleaved by separase; Hauf et al., 2001), provided that Scc1 has been isolated from mitotic cells (Noc, arrested with nocodazole). In contrast, the 896 (Scc1-2) antibodies can not react with Scc1-9xA, a form of Scc1 in which 9 serine and threonine residues have been mutated to alanine residues, including serine 46. These data indicate that the reactivity of the 986 antibodies with Scc1 depend on phosphorylation of serine 46 in Scc1.

Figure 11 shows that antibodies raised against mitosis specific phosphorylation sites on the cohesin subunits Scc1 and SA2 only react with cohesin isolated from mitotic cells: Cohesin complexes were isolated from human HeLa cells that had either been arrested in interphase (i) by treatment with hydroxyurea, or in mitosis (m) by treatment with nocodazole. Cohesin subunits were separated by SDS-PAGE and analyzed by immunoblotting with the antibodies that were raised against the phosphorylated cohesin peptides Scc1-1, Scc1-2, SA2-1 and SA2-2. Note that the antibodies only react with cohesin isolated from mitotic cells where the Scc1 and SA2 subunits are phosphorylated.

Figure 12 shows that antibodies raised against mitosis specific phosphorylation sites on the cohesin subunits Scc1 and SA2 can react with cohesin isolated from interphase cells after phosphorylation of cohesin by Plk1: Cohesin complexes were isolated from human HeLa cells that had either been arrested in interphase (i) by treatment with hydroxyurea, or in mitosis (m) by treatment with nocodazole. In one case the isolation of cohesin from mitotic cells was performed in buffers containing inhibitors of protein phosphatases (PPase inh.) and EDTA to stabilize phospho-sites on cohesin. In another case cohesin purified from interphase cells was incubated in buffers containing MgCl₂ and ATP in either the absence or presence of recombinant active Plk1. In all cases cohesin subunits were separated by SDS-PAGE and analyzed by immunoblotting with the antibodies that were raised against the phosphorylated cohesin peptides 896 (Scc1-2) and 897 (SA2-1). Note that the antibodies only react with cohesin isolated from interphase cells if cohesin has been phosphorylated by Plk1. It can also be seen that the 896 (Scc1-2) antibody reacts better with Scc1 from mitotic cells if phosphatases have been inhibited during the cohesin isolation procedure.

Figure 13 shows that the reactivity of phospho-specific antibodies with Scc1 is abolished by mutation of the phosphorylation site against which the antibodies were raised: Antibodies 896 (Scc1-2) were raised against mitosis specific phosphorylation on serine 46 of the cohesin subunit Scc1. To test the specificity of these antibodies cohesin complexes were isolated from HeLa cells that either contain forms of Scc1 in which serine 46 is present (Scc1-wt, Scc1-S454A, Scc1-NC), or forms of Scc1 in which serine 46 has been mutated to alanine (Scc1-9xA). All complexes were isolated from HeLa cells that had either been arrested in interphase by treatment with hydroxyurea (HU) or in mitosis by treatment with nocodazole (Noc). In some cases identical forms of the cohesin complexes were isolated from different HeLa cell lines (Scc1-S454A#1 and Scc1-S454A#2; two cell lines transfected with Scc1-myc, wherein serine 454 was mutated to alanine; Scc1-9xA#1 and Scc1-9xA#2, two cell lines transfected with Scc1-myc wherein all 9 phosphosites were mutated to alanine). In all cases cohesin subunits were separated by SDS-PAGE and analyzed by immunoblotting with the antibodies that were raised against the phosphorylated cohesin peptide 896 (Scc 1-2). Note that the antibodies only react with those forms of Scc1 which contain serine 46 and which have been isolated from mitotic cells. As a loading control, the samples were also analyzed by immunoblotting with antibodies to the myc epitope which is fused to Scc1 and with antibodies against the ccohesin subunits Smc1 and Smc3.

### References

Alexandru G, Uhlmann F, Mechtler K, Poupart M, Nasmyth K (2001) Phosphorylation of the cohesin subunit scc1 by polo/cdc5 kinase regulates sister chromatid separation in yeast. Cell 105(4): 459-472.
Barr FA, Sillje HH, Nigg EA (2004) Polo-like kinases and the orchestration of cell division. Nat Rev Mol Cell Biol 5(6): 429-440.
Birkenbihl RP, Subramani S (1995) The rad21 gene product of Schizosaccharomyces pombe is a nuclear, cell cycle-regulated phosphoprotein. J Biol Chem 270(13): 7703-7711.
Bulaj, G, Kortemme, T. and Goldenberg, D. P. (1998). Ionization-reactivity relationships for cysteine thiols in polypeptides. Biochemistry 37, 8965-8972.
Chan RC, Chan A, Jeon M, Wu TF, Pasqualone D et al. (2003) Chromosome cohesion is regulated by a clock gene paralogue TIM-1. Nature 423(6943): 1002-1009.
Ciosk R, Zachariae W, Michaelis C, Shevchenko A, Mann M et al. (1998). An ESP 1/PDS 1, complex regulates loss of sister chromatid cohesion at the metaphase to anaphase transition in yeast. Cell 93(6): 1067-1076.
Ciosk R, Shirayama M, Shevchenko A, Tanaka T, Toth A et al. (2000). Cohesin's binding to chromosomes depends on a separate complex consisting of Scc2 and Scc4 proteins. Mol Cell 5(2): 243-254.
Cohen-Fix O, Peters JM, Kirschner MW, Koshland D (1996). Anaphase initiation in Saccharomyces cerevisiae is controlled by the APC-dependent degradation of the anaphase inhibitor Pdslp. Genes Dev 10(24): 3081-3093.
Druker and Lydon, J Clin Invest. (2000) Jan;105(1):3-7.
Elia AE, Cantley LC, Yaffe MB (2003). Proteomic screen finds pSer/pThr-binding domain localizing Plk1 to mitotic substrates. Science 299(5610): 1228-1231.
Funabiki H, Yamano H, Kumada K, Nagao K, Hunt T et al. (1996) Cut2 proteolysis required for sister-chromatid separation in fission yeast. Nature 381(6581): 438-41 1
Fry, D.W., Bedford, D.C., Harvey, P.H.,Fritsch, A., Keller, P.R., Wu, Z., Dobrusin, E., Leopold, W.R., Fattaey (2001) J Biol Chem 276, 16617-16623.
Giménez-Abian JF, Sumara I, Hirota T, Hauf S, Gerlich D et al. (2004). Regulation of Sister Chromatid Cohesion between Chromosome Arms. Curr Biol. 2004 Jul 13; 14(13):1187-93.
Graves, P.R., Yu, L., Schwarz, J.K., Gales, J., Sausville, E.A., O'Connor, P.M., Piwnica-Worms, H. (2000). J. Biol. Chem 275, 5600-5605.
Gruber S, Haering CH, Nasmyth K (2003). Chromosomal cohesin forms a ring. Cell 112(6): 765-777.
Guacci V, Koshland D, Strunnikov A (1997). A direct link between sister chromatid cohesion and chromosome condensation revealed through the analysis of MCD1 in S. cerevisiae. Cell 91(1): 47-57.
Haering CH, Nasmyth K (2003). Building and breaking bridges between sister chromatids. Bioessays 25(12): 1178-1191.
Haering CH, Lowe J, Hochwagen A, Nasmyth K (2002). Molecular architecture of SMC proteins and the yeast cohesin complex. Mol Cell 9(4): 773-788.
Harlow, E. and Lane, D. (Eds.), (1988). Antibodies. A laboratory manual. Cold Spring Harbor laboratory Press. N.Y.
Hauf S, Waizenegger IC, Peters JM (2001). Cohesin cleavage by separase required for anaphase and cytokinesis in human cells. Science 293(5533): 1320-1323.
Hauf S, Cole RW, LaTerra S, Zimmer C, Schnapp G et al. (2003). The small molecule Hesperadin reveals a role for Aurora B in correcting kinetochore-microtubule attachment and in maintaining the spindle assembly checkpoint. J Cell Biol 161(2): 281-294.
Hoque MT, Ishikawa F (2001). Human chromatid cohesin component hRad21 is phosphorylated in M phase and associated with metaphase centromeres. J Biol Chem 276(7): 5059-5067.
Jallepalli PV, Waizenegger IC, Bunz F, Langer S, Speicher MR et al. (2001). Securin is required for chromosomal stability in human cells. Cell 105(4): 445-457.
Katis VL, Galova M, Rabitsch KP, Gregan J, Nasmyth K (2004). Maintenance of cohesin at centromeres after meiosis I in budding yeast requires a kinetochore-associated protein related to MEI-S332. Curr Biol 14(7): 560-572.
Kennet, (1980)."Monoclonal Antibodies, Hybridomas--A New Dimensionin Biological Analysis", Eds. Kennet, McKern and Bechtol, Plenum Press, N.Y. (1980).
Kerrebrock AW, Moore DP, Wu JS, Orr-Weaver TL (1995). Mei-S332, a Drosophila protein required for sister-chromatid cohesion, can localize to meiotic centromere regions. Cell 83(2): 247-256.
Kim ST, Xu B, Kastan MB (2002). Involvement of the cohesin protein, Smcl, in Atm-dependent and independent responses to DNA damage. Genes Dev 16(5): 560-570.
Kitada et al., Mol. Cell. Biol. 1993, 13: 4445-4457;
Kitajima TS, Kawashima SA, Watanabe Y (2004). The conserved kinetochore protein shugoshin protects centromeric cohesion during meiosis. Nature 427(6974): 510-517.
Kohler and Milstein, (1975), Nature 256: 495.
Kraft C, Herzog F, Gieffers C, Mechtler K, Hagting A et al. (2003). Mitotic regulation of the human anaphase-promoting complex by phosphorylation. Embo J 22(24): 6598-6609:
Lane and Nigg, J. Cell Biol. 1996, 135: 1701-1713.
Lewis at al. (1994). THE JOURNAL OF BIOLOGICAL CHEMISTRY, Vol. 269, No. 42, Issue of October 21, pp. 26259-26266.
Llamazares et al., Genes Dev. 1991, 5: 2153-2165.
Liu and Erikson, PNAS 2002, 99: 8672-8676.
Liu and Erikson, PNAS 2003, 100: 5789-5794.
Losada A, Hirano M, Hirano T (2002). Cohesin release is required for sister chromatid resolution, but not for condensin-mediated compaction, at the onset of mitosis. Genes Dev 16(23): 3004-3016.
Losada A, Yokochi T, Kobayashi R, Hirano T (2000). Identification and characterization of SA/Scc3p subunits in the Xenopus and human cohesin complexes. J Cell Biol 150(3): 405-416.
Marston AL, Tham WH, Shah H, Amon A (2004). A genome-wide screen identifies genes required,for centromeric cohesion. Science 303(5662): 1367-1370.
Michaelis C, Ciosk R, Nasmyth K (1997). Cohesins: chromosomal proteins that prevent premature separation of sister chromatids. Cell 91(1): 35-45.
Moore DP, Page AW, Tang TT, Kerrebrock AW, Orr-Weaver TL (1998). The cohesion protein MEI-S332 localizes to condensed meiotic and mitotic centromeres until sister chromatids separate. J Cell Biol 140(5): 1003-1012.
Moshe Y, Boulaire J, Pagano M, Hershko A (2004). Role of Polo-like kinase in the degradation of early mitotic inhibitor 1, a regulator of the anaphase promoting complex/cyclosome. Proc Natl Acad Sci U S A 101(21): 7937-7942.
Nakajima H, Toyoshima-Morimoto F, Taniguchi E, Nishida E (2003). Identification of a consensus motif for Plk (Polo-like kinase) phosphorylation reveals Myt1 as a Plk1 substrate. J Biol Chem 278(28): 25277-25280.
Peters JM (2002). The anaphase-promoting complex: proteolysis in mitosis and beyond. Mol Cell 9(5): 931-943.
Petronczki M, Siomos MF, Nasmyth K (2003). Un menage a quatre: the molecular biology of chromosome segregation in meiosis. Cell 112(4): 423-440.
Pezzi N, Prieto I, Kremer L, Perez Jurado LA, Valero C et al. (2000). STAG3, a novel gene encoding a protein involved in meiotic chromosome pairing and location of STAG3-related genes flanking the Williams-Beuren syndrome deletion. Faseb J 14(3): 581-592.
Rabitsch KP, Gregan J, Schleiffer A, Javerzat JP, Eisenhaber F et al. (2004). Two fission yeast homologs of Drosophila Mei-S332 are required for chromosome segregation during meiosis I and II. Curr Biol 14(4): 287-301.
Rena et al. (1999). J Biol Chem, June 11, Vol. 274, Issue 24, 17179-17183.
Rodriguez EM, Parra MT, Rufas JS, Suja JA (2001). Colchicine promotes a change in chromosome structure without loss of sister chromatid cohesion in prometaphase I-arrested bivalents. Chromosoma 110(7): 478-486.
Shou W, Azzam R, Chen SL, Huddleston MJ, Baskerville C et al. (2002). Cdc5 influences phosphorylation of Net1 and disassembly of the RENT complex. BMC Mol Biol 3(1): 3.
Spänkuch-Schmitt et al., Oncogene 2002, 21: 3162-3171.
Stemmann O, Zou H, Gerber SA, Gygi SP, Kirschner MW (2001). Dual inhibition of sister chromatid separation at metaphase. Cell 107(6): 715-726.
Sumara I, Vorlaufer E, Gieffers C, Peters BH, Peters JM (2000). Characterization of vertebrate cohesin complexes and their regulation in prophase. J Cell Biol 151(4): 749-762.
Sumara I, Vorlaufer E, Stukenberg PT, Kelm O, Redemann N et al. (2002). The dissociation of cohesin from chromosomes in prophase is regulated by Polo-like kinase. Mol Cell 9(3): 515-525.
Sumara et al., Curr. Biol. 2004, 14: 1712-1722).
Tomonaga T, Nagao K, Kawasaki Y, Furuya K, Murakami A et al. (2000). Characterization of fission yeast cohesin: essential anaphase proteolysis of Rad21 phosphorylated in the S phase. Genes Dev 14(21): 2757-2770.
Uhlmann F (2004). The mechanism of sister chromatid cohesion. Exp Cell Res 296(1): 80-85.
Uhlmann F, Lottspeich F, Nasmyth K (1999). Sister-chromatid separation at anaphase onset is promoted by cleavage of the cohesin subunit Scc1. Nature 400(6739): 37-42.
Uhlmann F, Wernic D, Poupart MA, Koonin EV, Nasmyth K (2000). Cleavage of cohesin by the CD clan protease separin triggers anaphase in yeast. Cell 103(3): 375-386.
Waizenegger IC, Hauf S, Meinke A, Peters JM (2000). Two distinct pathways remove mammalian cohesin from chromosome arms in prophase and from centromeres in anaphase. Cell 103(3): 399-410.
Wang, Y, Li, J., Booher, R.N., Kraker, A., Lawrence, T., Leopold, W.R. and Sun, Y (2001). Cancer Res 61, 8211-8217.
Wisdom, GB., (1994), Peptide Antigens, A Practical Approach, Oxford University Press, Eds. Rickwood D. And Hames B.D.
Yates JR, 3rd, Link AJ, Schieltz D (2000). Direct analysis of proteins in mixtures. Application to protein complexes. Methods Mol Biol 146: 17-26.
Yazdi PT, Wang Y, Zhao S, Patel N, Lee EY et al. (2002). SMC1 is a downstream effector in the ATM/NBS1 branch of the human S-phase checkpoint. Genes Dev 16(5): 571-582.
Yeong FM, Hombauer H, Wendt KS, Hirota T, Mudrak I et al. (2003). Identification of a subunit of a novel Kleisin-beta/SMC complex as a potential substrate of protein phosphatase 2A. Curr Biol 13(23): 2058-2064.
Yuan, J., Horlin, A., Hock, B., Stutte, HJ., Rubsamen-Waigmann, H., and Strebhardt, K. (1997). American Journal of Pathology, Apr;150(4):1165-72.
Zou H, McGarry TJ, Bernal T, Kirschner MW (1999). Identification of a vertebrate sister-chromatid separation inhibitor involved in transformation and tumorigenesis. Science 285(5426): 418-422.

## Claims

1. An antibody that binds to a phosphorylation site of the human cohesin subunit Scc1 (SEQ ID NO:1) or of the human cohesin subunit SA2 (SEQ ID NO:2) when said phosphorylation site of Scc1 or SA2 is phosphorylated and that does not bind to the potential phosphorylation site when said potential phosphorylation site is not phosphorylated.

2. The antibody of claim 1 that binds to a phosphorylation site selected from serine 46, serine 138, threonine 144, serine 153, serine 175, threonine 312, serine 454, serine 459 or serine 466 of SEQ ID NO:1.

3. The antibody of claim 1 that binds to a phosphorylation site selected from serine 185, threonine 186, threonine 187, threonine 188 and serine 189 of SEQ ID NO: 1

4. The antibody of claim 1 that binds to a phosphorylation site selected from serine 843, serine 1058, threonine 1118, threonine 1146, threonine 1151, threonine 1193 or serine 1224 of SEQ ID NO:2.

5. The antibody of claim 1 that -binds to a phosphorylation site selected from serine 377 and threonine 379 of SEQ ID NO:2.

6. The antibody of claim 1 that binds to a phosphorylation site selected from serine 1064 and serine 1065 of SEQ ID NO:2.

7. The antibody of claim 1 that binds to a phosphorylation site selected from threonine 1109 and threonine 1112 of SEQ ID NO:2.

8. The antibody of claim 1 that binds to a phosphorylation site selected from serine 1123 and threonine 1124 of SEQ ID NO:2.

9. The antibody of claim 1 that binds to a phosphorylation site selected from serine 1137 and serine 1140 of SEQ ID NO:2.

10. The antibodyof claim 1- that binds to a phosphorylation site selected from threonine 1160 and serine 1161 of SEQ ID NO:2.

11. The antibody of claim 1 that binds to a phosphorylation site selected from serine 1177 and serine 1178 of SEQ ID NO:2.

12. The antibody of any one of claims 1 to 11, which is a monoclonal antibody.

13. An immunogenic composition comprising an antigenic phospho-peptide derived from a polypeptide selected from SEQ ID NO:1 or SEQ ID NO:2.

14. A method for detecting Plkl activity in a cell *ex vivo* or *in vitro,* wherein the presence of phosphorylated Scc1 and/or SA2 is determined by
a. contacting a biological sample suspected of Plk1 activity with one or more phospho-specific antibodies of any one of claims 1 to 12 and
b. determining binding of said antibody or antibodies to Scc1 and/or SA2, wherein the amount of bound antibody is indicative of the level of Plkl activity.

15. A method for determining a compound's effect on Plkl activity in an individual treated with said compound by determining *ex vivo,* before and after administration of said compound to said individual, the presence of phosphorylated Scc1 and/or SA2 in a sample, wherein
a. a sample obtained from said individual is contacted with one or more antibodies of any one of claims 1 to 12 and
b. binding of said antibody or antibodies to Scc1 and/or SA2 is determined, wherein the amount of bound antibody or antibodies is indicative of the level of Plk1 activity,
c. the levels of phosphorylated Scc1 and/or SA2 before and after administration of said compound are compared,
wherein a reduced level of phosphorylated Scc1 and/or SA2 upon administration of said compound is indicative of its inhibitory effect on Plk1.

16. The method of claim 15, wherein said compound's effect on Plk1 activity is determined over a period of time at pre-defined time points by comparing the levels of phosphorylated Scc1 and/or SA2 over said period of time,
wherein a reduced level of phosphorylated Scc1 and/or SA2 is indicative of said compound's effect on Plk1 activity.

17. A method for selecting a cancer patient for being treated with a compound that acts as inhibitor of Plk1, by determining *ex vivo,* the presence of phosphorylated Scc1 and/or SA2 in cells of said patient, wherein
a. a sample obtained from a tumor of said patient is contacted with one or more antibodies of any one of claims 1 to 12 and
b. binding of said antibody or antibodies to Scc1 and/or SA2 is determined,
wherein a high level of Scc1 and/or SA2 phosphorylation is indicative of aberrant Plkl activity and a patient exhibiting such high phosphorylation level is selected for treatment with said Plkl inhibitory compound.

18. The use of an antibody in any one of claims 1 to 11 in a method for determining or confirming a compound's ability to inhibit Plk1 in a cell,
wherein
a. a population of cells having Plk1 activity is adhered to a first solid support and contacted with a candidate compound for a period of time sufficient for the compound to exert its inhibitory effect on Plk1,
b. a capture antibody that binds to Scc1 or SA2 at a site different from a phosphorylation site is adhered to a second solid support,
c. a lysate of cells obtained from step a. is added to said second solid support to capture Scc1 or SA2, respectively,
d. unbound cell lysate is removed from the second solid support,
e. the captured Scc1 or SA2 is exposed to a antibody of any one of claims 1 to 11 that carries a detectable label,
f. binding of said labeled antibody to the captured Scc1 or SA2 is measured,
g. the signal obtained in step f. is compared with the signal obtained from control cells obtained without exposure of compound, wherein a reduction of the signal as compared to the control cells is indicative of said compound's inhibitory effect on Plk1.


SEQUENCE LISTING

<110>  Boehringer Ingelheim International GmbH

<120>  Surrogate Markers for Plk1 activity

<130>  Case 14-0085

<160>  3

<170>  PatentIn version 3.1

<210>  1

<211>  631

<212>  PRT

<213>  Homo sapiens

<400>  1

<210> 2

<211> 1231

<212> PRT

<213> Homo sapiens

<400> 2

Met Ile Ala Ala Pro Glu Ile Pro Thr Asp Phe Asn Leu Leu Gln Glu
1               5                   10                  15

Ser Glu Thr His Phe Ser Ser Asp Thr Asp Phe Glu Asp Ile Glu Gly
            20                  25                  30

Lys Asn Gln Lys Gln Gly Lys Gly Lys Thr Cys Lys Lys Gly Lys Lys
        35                  40                  45

Gly Pro Ala Glu Lys Gly Lys Gly Gly Asn Gly Gly Gly Lys Pro Pro
    50                  55                  60

Ser Gly Pro Asn Arg Met Asn Gly His His Gln Gln Asn Gly Val Glu
65                  70                  75                  80

Asn Met Met Leu Phe Glu Val Val Lys Met Gly Lys Ser Ala Met Gln
                85                  90                  95

Ser Val Val Asp Asp Trp Ile Glu Ser Tyr Lys His Asp Arg Asp Ile
            100                 105                 110

Ala Leu Leu Asp Leu Ile Asn Phe Phe Ile Gln Cys Ser Gly Cys Lys
        115                 120                 125

Gly Val Val Thr Ala Glu Met Phe Arg His Met Gln Asn Ser Glu Ile
    130                 135                 140

Ile Arg Lys Met Thr Glu Glu Phe Asp Glu Asp Ser Gly Asp Tyr Pro
145                 150                 155                 160

Leu Thr Met Ala Gly Pro Gln Trp Lys Lys Phe Lys Ser Ser Phe Cys
                165                 170                 175

Glu Phe Ile Gly Val Leu Val Arg Gln Cys Gln Tyr Ser Ile Ile Tyr
            180                 185                 190

Asp Glu Tyr Met Met Asp Thr Val Ile Ser Leu Leu Thr Gly Leu Ser
        195                 200                 205

Asp Ser Gln Val Arg Ala Phe Arg His Thr Ser Thr Leu Ala Ala Met
    210                 215                 220

Lys Leu Met Thr Ala Leu Val Asn Val Ala Leu Asn Leu Ser Ile Asn
225                 230                 235                 240

Met Asp Asn Thr Gln Arg Gln Tyr Glu Ala Glu Arg Asn Lys Met Ile
                245                 250                 255

Gly Lys Arg Ala Asn Glu Arg Leu Glu Leu Leu Leu Gln Lys Arg Lys
            260                 265                 270

Glu Leu Gln Glu Asn Gln Asp Glu Ile Glu Asn Met Met Asn Ala Ile
        275                 280                 285

Phe Lys Gly Val Phe Val His Arg Tyr Arg Asp Ala Ile Ala Glu Ile
    290                 295                 300

Arg Ala Ile Cys Ile Glu Glu Ile Gly Ile Trp Met Lys Met Tyr Ser
305                 310                 315                 320

Asp Ala Phe Leu Asn Asp Ser Tyr Leu Lys Tyr Val Gly Trp Thr Met
                325                 330                 335

His Asp Lys Gln Gly Glu Val Arg Leu Lys Cys Leu Thr Ala Leu Gln
            340                 345                 350

Gly Leu Tyr Tyr Asn Lys Glu Leu Asn Ser Lys Leu Glu Leu Phe Thr
        355                 360                 365

Ser Arg Phe Lys Asp Arg Ile Val Ser Met Thr Leu Asp Lys Glu Tyr
    370                 375                 380

Asp Val Ala Val Gln Ala Ile Lys Leu Leu Thr Leu Val Leu Gln Ser
385                 390                 395                 400

Ser Glu Glu Val Leu Thr Ala Glu Asp Cys Glu Asn Val Tyr His Leu
                405                 410                 415

Val Tyr Ser Ala His Arg Pro Val Ala Val Ala Ala Gly Glu Phe Leu
            420                 425                 430

Tyr Lys Lys Leu Phe Ser Arg Arg Asp Pro Glu Glu Asp Gly Met Met
        435                 440                 445

Lys Arg Arg Gly Arg Gln Gly Pro Asn Ala Asn Leu Val Lys Thr Leu
    450                 455                 460

Val Phe Phe Phe Leu Glu Ser Glu Leu His Glu His Ala Ala Tyr Leu
465                 470                 475                 480

Val Asp Ser Met Trp Asp Cys Ala Thr Glu Leu Leu Lys Asp Trp Glu
                485                 490                 495

Cys Met Asn Ser Leu Leu Leu Glu Glu Pro Leu Ser Gly Glu Glu Ala
            500                 505                 510

Leu Thr Asp Arg Gln Glu Ser Ala Leu Ile Glu Ile Met Leu Cys Thr
        515                 520                 525

Ile Arg Gln Ala Ala Glu Cys His Pro Pro Val Gly Arg Gly Thr Gly
    530                 535                 540

Lys Arg Val Leu Thr Ala Lys Glu Lys Lys Thr Gln Leu Asp Asp Arg
545                 550                 555                 560

Thr Lys Ile Thr Glu Leu Phe Ala Val Ala Leu Pro Gln Leu Leu Ala
                565                 570                 575

Lys Tyr Ser Val Asp Ala Glu Lys Val Thr Asn Leu Leu Gln Leu Pro
            580                 585                 590

Gln Tyr Phe Asp Leu Glu Ile Tyr Thr Thr Gly Arg Leu Glu Lys His
        595                 600                 605

Leu Asp Ala Leu Leu Arg Gln Ile Arg Asn Ile Val Glu Lys His Thr
    610                 615                 620

Asp Thr Asp Val Leu Glu Ala Cys Ser Lys Thr Tyr His Ala Leu Cys
625                 630                 635                 640

Asn Glu Glu Phe Thr Ile Phe Asn Arg Val Asp Ile Ser Arg Ser Gln
                645                 650                 655

Leu Ile Asp Glu Leu Ala Asp Lys Phe Asn Arg Leu Leu Glu Asp Phe
            660                 665                 670

Leu Gln Glu Gly Glu Glu Pro Asp Glu Asp Asp Ala Tyr Gln Val Leu
        675                 680                 685

Ser Thr Leu Lys Arg Ile Thr Ala Phe His Asn Ala His Asp Leu Ser
    690                 695                 700

Lys Trp Asp Leu Phe Ala Cys Asn Tyr Lys Leu Leu Lys Thr Gly Ile
705                 710                 715                 720

Glu Asn Gly Asp Met Pro Glu Gln Ile Val Ile His Ala Leu Gln Cys
                725                 730                 735

Thr His Tyr Val Ile Leu Trp Gln Leu Ala Lys Ile Thr Glu Ser Ser
            740                 745                 750

Ser Thr Lys Glu Asp Leu Leu Arg Leu Lys Lys Gln Met Arg Val Phe
        755                 760                 765

Cys Gln Ile Cys Gln His Tyr Leu Thr Asn Val Asn Thr Thr Val Lys
    770                 775                 780

Glu Gln Ala Phe Thr Ile Leu Cys Asp Ile Leu Met Ile Phe Ser His
785                 790                 795                 800

Gln Ile Met Ser Gly Gly Arg Asp Met Leu Glu Pro Leu Val Tyr Thr
                805                 810                 815

Pro Asp Ser Ser Leu Gln Ser Glu Leu Leu Ser Phe Ile Leu Asp His
            820                 825                 830

Val Phe Ile Glu Gln Asp Asp Asp Asn Asn Ser Ala Asp Gly Gln Gln
        835                 840                 845

Glu Asp Glu Ala Ser Lys Ile Glu Ala Leu His Lys Arg Arg Asn Leu
    850                 855                 860

Leu Ala Ala Phe Cys Lys Leu Ile Val Tyr Thr Val Val Glu Met Asn
865                 870                 875                 880

Thr Ala Ala Asp Ile Phe Lys Gln Tyr Met Lys Tyr Tyr Asn Asp Tyr
                885                 890                 895

Gly Asp Ile Ile Lys Glu Thr Met Ser Lys Thr Arg Gln Ile Asp Lys
            900                 905                 910

Ile Gln Cys Ala Lys Thr Leu Ile Leu Ser Leu Gln Gln Leu Phe Asn
        915                 920                 925

Glu Met Ile Gln Glu Asn Gly Tyr Asn Phe Asp Arg Ser Ser Ser Thr
    930                 935                 940

Phe Ser Gly Ile Lys Glu Leu Ala Arg Arg Phe Ala Leu Thr Phe Gly
945                 950                 955                 960

Leu Asp Gln Leu Lys Thr Arg Glu Ala Ile Ala Met Leu His Lys Asp
                965                 970                 975

Gly Ile Glu Phe Ala Phe Lys Glu Pro Asn Pro Gln Gly Glu Ser His
            980                 985                 990

Pro Pro Leu Asn Leu Ala Phe Leu  Asp Ile Leu Ser Glu  Phe Ser Ser
        995                 1000                 1005

Lys Leu  Leu Arg Gln Asp Lys  Arg Thr Val Tyr Val  Tyr Leu Glu
    1010                 1015                 1020

Lys Phe Met Thr Phe Gln Met Ser Leu Arg Arg Glu Asp Val Trp
    1025                1030                1035

Leu Pro Leu Met Ser Tyr Arg Asn Ser Leu Leu Ala Gly Gly Asp
    1040                1045                1050

Asp Asp Thr Met Ser Val Ile Ser Gly Ile Ser Ser Arg Gly Ser
    1055                1060                1065

Thr Val Arg Ser Lys Lys Ser Lys Pro Ser Thr Gly Lys Arg Lys
    1070                1075                1080

Val Val Glu Gly Met Gln Leu Ser Leu Thr Glu Glu Ser Ser Ser
    1085                1090                1095

Ser Asp Ser Met Trp Leu Ser Arg Glu Gln Thr Leu His Thr Pro
    1100                1105                1110

Val Met Met Gln Thr Pro Gln Leu Thr Ser Thr Ile Met Arg Glu
    1115                1120                1125

Pro Lys Arg Leu Arg Pro Glu Asp Ser Phe Met Ser Val Tyr Pro
    1130                1135                1140

Met Gln Thr Glu His His Gln Thr Pro Leu Asp Tyr Asn Arg Arg
    1145                1150                1155

Gly Thr Ser Leu Met Glu Asp Asp Glu Glu Pro Ile Val Glu Asp
    1160                1165                1170

Val Met Met Ser Ser Glu Gly Arg Ile Glu Asp Leu Asn Glu Gly
    1175                1180                1185

Met Asp Phe Asp Thr Met Asp Ile Asp Leu Pro Pro Ser Lys Asn
    1190                1195                1200

Arg Arg Glu Arg Thr Glu Leu Lys Pro Asp Phe Phe Asp Pro Ala
    1205                1210                1215

Ser Ile Met Asp Glu Ser Val Leu Gly Val Ser Met Phe
    1220                1225                1230

<210>  3

<211>  21

<212>  PRT

<213>  Homo sapiens

<400>  3

Thr Asp Gln Ala Asp Gly Pro Arg Glu Pro Pro Gln Ser Ala Arg Arg
1               5                   10                  15

Lys Arg Ser Tyr Cys
            20